# EUROPÄISCHE PATENTANMELDUNG

(11) **EP 0 685 466 A1**
(43) Veröffentlichungstag der Anmeldung: **06.12.1995**
(21) Anmeldenummer: 95810344.2
(22) Anmeldetag: 23.05.1995
(51) Int. Cl.: C07D 215/36, C07D 215/22, C07D 401/12, C07D 409/12, C07D 405/12, A61K 31/475, A61K 31/505

(54) **3-Heteroaliphatyl- und 3-Hetero-(aryl)aliphatyl-2(1H)-chinolonderivate**

(30) Priorität: 02.06.1994 CH 1732/94
(71) Anmelder: CIBA-GEIGY AG, CH-4002 Basel (CH)
(72) Erfinder: Allgeier, Hans, Dr., D-79541 Lörrach (DE)

(57) **Zusammenfassung**

3-Heteroaliphatyl- und 3-Hetero(aryl)aliphatyl2(1H)chinolonderivate der Formel I 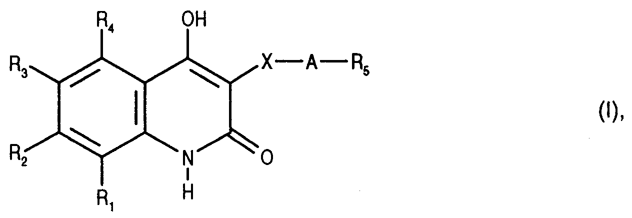
worin die Reste R₁, R₂, R₃ und R₄ unabhängig voneinander Wasserstoff, aliphatische Kohlenwasserstoffreste, gegebenenfalls verethertes Hydroxy, gegebenenfalls verethertes und/oder oxidiertes Mercapto, unsubstituiertes oder aliphatisch substituiertes Amino, Nitro, gegebenenfalls verestertes oder amidiertes Carboxy, Cyano, gegebenenfalls amidiertes Sulfamoyl, Halogen oder Trifluormethyl bedeuten, X Oxy oder gegebenenfalls oxidiertes Thio darstellt, A für einen zweiwertigen aliphatischen Rest steht und R₅ R₅ einen unsubstituierten oder durch aliphatische oder araliphatische Kohlenwasserstoffreste, gegebenenfalls verethertes Hydroxy, gegebenenfalls verethertes und/oder oxidiertes Mercapto, unsubstituiertes oder aliphatisch substituiertes Amino, aliphatisches Acyl, gegebenenfalls verestertes oder amidiertes Carboxy, Cyano, gegebenenfalls amidiertes Sulfamoyl, Halogen und/oder Trifluormethyl substituierten, gegebenenfalls partiell hydrierten Aryl- oder Hetroarylrest, gegebenenfalls. verethertes Hydroxymethyl, Cyano oder gegebenenfalls verestertes oder amidiertes Carboxy darstellt, darstellt, ihre Tautomeren und/oder Salze, weisen antagonistische Eigenschaften gegenüber excitatorischen Aminosäuren auf und können als antikonvulsive und anti-neurodegenerative Arzneimittelwirkstoffe verwendet werden.

## Beschreibung

Die Erfindung betrifft neue 3-Heteroaliphatyl- und 3-Hetero(aryl)aliphatyl-2(1 H)-chinolon-derivate der Formel I 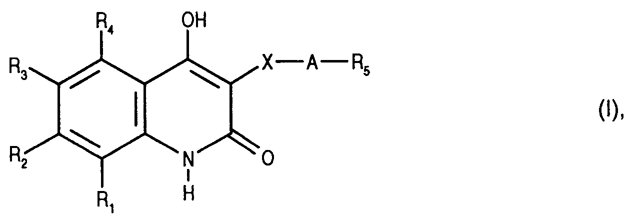
worin
die Reste R₁, R₂, R₃ und R₄ unabhängig voneinander Wasserstoff, aliphatische Kohlenwasserstoffreste, gegebenenfalls verethertes Hydroxy, gegebenenfalls verethertes und/oder oxidiertes Mercapto, unsubstituiertes oder aliphatisch substituiertes Amino, Nitro, gegebenenfalls verestertes oder amidiertes Carboxy, Cyano, gegebenenfalls amidiertes Sulfamoyl, Halogen oder Trifluormethyl bedeuten,
X Oxy oder gegebenenfalls oxidiertes Thio darstellt,
A für einen zweiwertigen aliphatischen Rest steht und
R₅ einen unsubstituierten oder durch aliphatische oder araliphatische Kohlenwasserstoffreste, gegebenenfalls verethertes Hydroxy, gegebenenfalls verethertes und/oder oxidiertes Mercapto, unsubstituiertes oder aliphatisch substituiertes Amino, aliphatisches Acyl, gegebenenfalls verestertes oder amidiertes Carboxy, Cyano, gegebenenfalls amidiertes Sulfamoyl, Halogen und/oder Trifluormethyl substituierten, gegebenenfalls partiell hydrierten Aryl- oder Heteroarylrest, gegebenenfalls. verethertes Hydroxymethyl, Cyano oder gegebenenfalls verestertes oder amidiertes Carboxy darstellt,
ihre Tautomeren und/oder Salze, die neuen Verbindungen enthaltende pharmazeutische Präparate und ihre Verwendung als Arzneimittelwirkstoffe.

Aliphatische Kohlenwasserstoffreste sind beispielsweise Niederalkyl- oder Niederalkenylreste, können aber auch Niederalkinylreste sein.

Gegebenenfalls partiell hydrierte Arylreste sind beispielsweise monocyclische Arylreste, wie Phenyl, bicyclische Arylreste, wie Naphthyl oder Indenyl, oder partiell hydrierte, vorzugsweise über ein gesättigtes C-Atom gebundene, bicyclische Arylreste, wie 1,2,3,4-Tetrahydronaphth-1-yl, 1,2,3,4-Tetrahydronaphth-2-yl, Indan-1-yl oder Indan2-yl. Die genannten aromatische Reste können einen oder mehrere gleiche oder verschiedene der genannten Substituenten aufweisen, d.h. mono-, di- oder trisubstituiert sein.

Gegebenenfalls partiell hydrierte Heteroarylreste sind beispielsweise über ein C-Atom gebundene, als Heteroatom(e) 1, 2, 3 oder 4 N-Atome oder 1 O- oder S-Atom aufweisende monocyclische oder bicyclische Heteroarylreste, wie Pyrrolyl, Indolyl, Furyl, Benzfuranyl, Thienyl, Benzthienyl, Imidazolyl, Benzimidazolyl, Oxazolyl, Benzoxazoyl, Thiazolyl, Benzthiazolyl, Tetrazolyl, Pyridyl, Chinolinyl, Isochinolinyl, Pyrimidinyl, Chinazolinyl, Pyridazinyl, Cinnolinyl, Pyrazinyl, Chinoxalinyl oder Triazinyl, oder partiell hydrierte, vorzugsweise über ein gesättigtes C-Atom gebundene, 1 oder 2 O- oder S-Atome aufweisende bicyclische Heteroarylreste, wie Chroman-3-yl, Chroman-4-yl oder 1,4-Benzdioxan-2-yl. Die genannten aromatische Reste können einen oder mehrere gleiche oder verschiedene der genannten Substituenten aufweisen, d.h. mono-, di- oder trisubstituiert sein.

Gegebenenfalls verethertes Hydroxy ist beispielsweise Hydroxy oder Niederalkoxy, kann aber auch Niederalkenyloxy oder Niederalkinyloxy sein.

Gegebenenfalls. verethertes Hydroxymethyl ist beispielsweise Hydroxymethyl oder Phenyloxymethyl.

Gegebenenfalls verethertes und/oder oxidiertes verethertes Mercapto ist beispielsweise gegebenenfalls oxidiertes Mercapto oder Niederalkylthio, wie Mercapto, Sulfo, Niederalkylthio, Niederalkansulfinyl oder Niederalkansulfonyl, kann aber auch gegebenenfalls oxidiertes Niederalkenylthio oder Niederalkinylthio, wie Niederalkensulfonyl, sein.

Aliphatisch substituiertes Amino ist beispielsweise N-Mono- oder N,N-Diniederalkylamino.

Gegebenenfalls verestertes oder amidiertes Carboxy ist beispielsweise Carboxy, Niederalkoxycarbonyl, unsubstituiertes oder durch Niederalkyl, Niederalkoxy, Hydroxy, Halogen und/oder Trifluormethyl substituiertes Phenylniederalkoxycarbonyl, Carbamoyl, N-Mono- oder N,N-Diniederalkylcarbamoyl, oder jeweils unsubstituiertes oder im Phenylteil durch Niederalkyl, Niederalkoxy, Hydroxy, Halogen und/oder Trifluormethyl substituiertes N-Phenylcarbamoyl oder N-Phenyl-N-niederalkyl-carbamoyl.

Gegebenenfalls amidiertes Sulfamoyl ist beispielsweise Sulfamoyl oder N-Mono- oder N,N-Diniederalkylsulfamoyl.

Gegebenenfalls oxidiertes Thio ist Thio, Sulfinyl oder Sulfonyl.

Der zweiwertige aliphatische Rest A ist beispielsweise ein durch gegebenenfalls verestertes Carboxy, wie Carboxy oder Niederalkoxycarbonyl, oder in höherer als der α-Stellung zur Thiogruppe durch gegebenenfalls verethertes Hydroxy, wie Hydroxy, Niederalkoxy oder in zweiter Linie Niederalkenyloxy oder Niederalkinyloxy, substituierter Niederalkylenrest. Als solche Reste sind insbesondere zu nennen: Niederalkylen. Carboxyniederalkylen, Niederalkoxycarbonylniederalkylen, die Hydroxy-, Niederalkoxy-, Niederalkenyloxy- oder Niederalkinyloxygruppe in höherer als der α-Stellung tragendes Hydroxyniederalkylen, Niederalkoxyniederalkylen, Niederalkenyloxyniederalkylen und Niederalkinyloxyniederalkylen.

Aliphatisches Acyl ist beispielsweise Niederalkanoyl.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalkyl ist beispielsweise C₁-C₇-Alkyl, vorzugsweise C₁-C₄-Alkyl, wie insbesondere Methyl oder in zweiter Linie Ethyl, Propyl, Isopropyl oder Butyl, kann aber auch Isobutyl, Sekundärbutyl, Tertiärbutyl oder eine C₅-C₇-Alkyl-, wie Pentyl-, Hexyl- oder Heptylgruppe sein.

Niederalkenyl ist beispielsweise C₃-C₄-Alkenyl, wie Allyl oder Methallyl.

Niederalkinyl ist beispielsweise C₃-C₄-Alkinyl, wie Propargyl.

Niederalkoxy ist beispielsweise C₁-C₇-Alkoxy, vorzugsweise C₁-C₄-Alkoxy, wie Methoxy, Ethoxy, Propyloxy, Isopropyloxy oder Butyloxy, kann aber auch Isobutyloxy, Sekundärbutyloxy, Tertiärbutyloxy oder eine Pentyloxy-, Hexyloxy- oder Heptyloxygruppe sein.

Niederalkenyloxy ist beispielsweise C₃-C₄-Alkenyloxy, wie Allyloxy oder Methallyloxy.

Niederalkinyloxy ist beispielsweise C₃-C₄-Alkinyloxy, wie Propargyloxy.

Niederalkylthio ist beispielsweise C₁-C₇-Alkylthio, vorzugsweise C₁-C₄-Alkylthio, wie Methylthio, Ethylthio, Propylthio, Isopropylthio oder Butylthio, kann aber auch Isobutylthio, Sekundärbutylthio, Tertiärbutylthio oder eine Pentylthio-, Hexylthio- oder Heptylthiogruppe sein.

Niederalkansulfinyl ist beispielsweise C₁-C₇-Alkansulfinyl, vorzugsweise C₁-C₄-Alkansulfinyl, wie Methansulfinyl, Ethansulfinyl, Propansulfinyl oder Butansulfinyl , kann aber auch eine Pentansulfinyl-, Hexansulfinyl- oder Heptansulfinylgruppe sein.

Niederalkansulfonyl is beispielsweise C₁-C₇-Alkansulfonyl, vorzugsweise C₁-C₄-Alkansulfonyl, wie Methansulfonyl, Ethansulfonyl, Propansulfonyl oder Butansulfonyl, kann aber auch eine Pentansulfonyl-, Hexansulfonyl- oder Heptansulfonylgruppe sein.

Niederalkenylthio ist beispielsweise C₃-C₄-Alkenylthio, wie Allylthio oder Methallylthio.

Niederalkensulfinyl ist beispielsweise C₃-C₄-Alkensulfinyl, wie Prop-2-ensulfinyl oder But-2-enylsulfinyl.

Niederalkensulfonyl ist beispielsweise C₃-C₄-Alkensulfonyl, wie Prop-2-ensulfonyl oder But-2-enylsulfonyl.

Niederalkinylthio ist beispielsweise C₃-C₄-Alkinylthio, wie Propargylthio.

N-Mono- oder N,N-Diniederalkylamino ist beispielsweise N-Mono- oder N,N-Di-C₁-C₇-Alkylamino, vorzugsweise N-Mono- oder N,N-Di-C₁-C₄-Alkylamino, wie insbesondere Methylamino, Dimethylamino, oder in zweiter Linie Ethylamino, Diethylamino, Propylamino. Dipropylamino, Isopropylamino, Diisopropylamino, Butylamino oder Dibutylamino, kann aber auch Isobutylamino, Sekundärbutylamino, Tertiärbutylamino oder eine C₅-C₇-Alkylamino-, wie Pentylamino-, Hexylamino- oder Heptylaminogruppe sein.

Niederalkoxycarbonyl ist beispielsweise C₁-C₇-Alkoxycarbonyl, vorzugsweise C₁-C₄-Alkoxycarbonyl, wie Methoxycarbonyl, Ethoxycarbonyl, Propyloxycarbonyl, Isopropyloxycarbonyl oder Butyloxycarbonyl, kann aber auch Isobutyloxycarbonyl, Sekundärbutyloxycarbonyl, Tertiärbutyloxycarbonyl oder eine Pentyloxycarbonyl-, Hexyloxycarbonyl- oder Heptyloxycarbonylgruppe sein.

Phenylniederalkoxycarbonyl ist beispielsweise unsubstituiertes oder durch C₁-C₄-Alkyl, C₁-C₄-Alkoxy, Hydroxy, Halogen und/oder Trifluormethyl substituiertes Phenyl-C₁-C₄-alkoxycarbonyl, wie Benzyloxycarbonyl oder α-Phenylethoxycarbonyl.

N-Mono- oder N,N-Diniederalkylcarbamoyl ist beispielsweise N-Mono- oder N,N-Di-C₁-C₇-Alkylcarbamoyl, vorzugsweise N-Mono- oder N,N-Di-C₁-C₄-Alkylcarbamoyl, wie insbesondere Methylcarbamoyl, Dimethylcarbamoyl, oder in zweiter Linie Ethylcarbamoyl, Diethylcarbamoyl, Propylcarbamoyl, Dipropylcarbamoyl, Isopropylcarbamoyl, Diisopropylcarbamoyl, Butylcarbamoyl oder Dibutylcarbamoyl, kann aber auch Isobutylcarbamoyl, Sekundärbutylcarbamoyl, Tertiärbutylcarbamoyl oder eine C₅-C₇-Alkylcarbamoyl-, wie Pentylcarbamoyl-, Hexylcarbamoyl- oder Heptylcarbamoylgruppe sein.

N-Mono- oder N,N-Diniederalkylsulfamoyl ist beispielsweise N-Mono- oder N,N-Di-C₁-C₇-Alkylsulfamoyl, vorzugsweise N-Mono- oder N,N-Di-C₁-C₄-Alkylsulfamoyl, wie insbesondere Methylsulfamoyl, Dimethylsulfamoyl, oder in zweiter Linie Ethylsulfamoyl, Diethylsulfamoyl, Propylsulfamoyl. Dipropylsulfamoyl, Isopropylsulfamoyl, Diisopropylsulfamoyl, Butylsulfamoyl oder Dibutylsulfamoyl, kann aber auch Isobutylsulfamoyl, Sekundärbutylsulfamoyl, Tertiärbutylsulfamoyl oder eine C₅-C₇-Alkylsulfamoyl-, wie Pentylsulfamoyl-, Hexylsulfamoyl- oder Heptylsulfamoylgruppe sein.

Niederalkylen kann geradkettig oder verzweigt sowie in beliebiger Stellung gebunden sein und ist beispielsweise geradkettiges oder verzweigtes C₁-C₇-Alkylen, vorzugsweise C₁-C₄-Alkylen, wie Methylen, Ethylen, 1,3- oder 1,2- Propylen, 1,4-, 1,3- oder 2,3-Butylen oder in zweiter Linie 1,5-, 1,4- oder 2,5-Pentylen.

Niederalkenylen kann geradkettig oder verzweigt sowie in beliebiger Stellung gebunden sein und ist beispielsweise geradkettiges oder verzweigtes C₂-C₇-Alkenylen, insbesondere C₂-C₄-Alkylen, wie Vinylen, 1,3-Prop-2-enylen oder 1,4-Butyl-2-enylen,

Carboxyniederalkylen ist beispielsweise Carboxy-C₁-C₄-alkylen, wie Carboxymethylen, 1- oder 2-Carboxyethylen, 1,3-(1-, 2- oder 3-Carboxy)propylen, 1,4-(1-, 2-, 3- oder 4-Carboxy)butylen, kann aber auch eine Carboxypentylen- oder Carboxyhexylgruppe sein.

Niederalkoxycarbonylniederalkylen ist beispielsweise C₁-C₄-Alkoxycarbonyl-C₁-C₄-alkylen, wie C₁-C₄-Alkoxycarbonylmethylen, 1- oder 2-C₁-C₄-Alkoxycarbonylethylen, 1,3-(1-, 2- oder 3- C₁-C₄-Alkoxycarbonyl)propylen, 1,4-(1-, 2-, 3- oder 4- C₁-C₄-Alkoxycarbonyl)butylen, kann aber auch eine C₁-C₄-Alkoxycarbonylpentylen- oder C₁-C₄-Alkoxycarbonylhexylengruppe sein. C₁-C₄-Alkoxycarbonyl bedeutet dabei beispielsweise geradkettig-, Ethoxy-, Propyloxy-, Isopropyloxy- oder Butyloxycarbonyl.

Die Hydroxygruppe in höherer als der α-Stellung zur Thiogruppe tragendes Hydroxyniederalkylen ist beispielsweise entsprechendes Hydroxy-C₂-C₄-alkylen, wie 1,3-(2-Hydroxy)propylen, 1,3-(3-Hydroxy)propylen, 1,4-(2-Hydroxy)butylen, 1,4-(3-Hydroxy)butylen, 1,4-(4-Hydroxy)butylen, kann aber auch 1,5-(2-Hydroxy)pentylen, 1,5-(3-Hydroxy)pentylen, 1,5-(4-Hydroxy)pentylen oder 1,5-(5-Hydroxy)pentylen sein.

Die Niederalkoxygruppe in höherer als der α-Stellung tragendes Niederalkoxycarbonylniederalkylen ist beispielsweise C₁-C₄-Alkoxy-C₂-C₄-alkylen, wie 1,3-(2-C₁-C₄-Alkoxy)propylen,1,3-(3-C₁-C₄-Alkoxy)propylen, 1,4-(2-C₁-C₄-Alkoxy)butylen, 1,4-(3-C₁-C₄-Alkoxy)butylen, 1 ,4-(4-C₁-C₄-Alkoxy)butylen, kann aber auch 1,5-(2-C₁-C₄-Alkoxy)pentylen, 1,5-(3-C₁-C₄-Alkoxy)pentylen, 1,5-(4-C₁-C₄-Alkoxy)pentylen oder 1,5-(5-C₁-C₄-Alkoxy)pentylen sein. C₁-C₄-Alkoxy hat dabei beispielsweise eine der vorstehenden Bedeutungen und ist insbesondere Methoxy oder Ethoxy.

Die Niederalkenyloxy- oder Niederalkinyloxygruppe in höherer als der α-Stellung tragendes Niederalkenyloxyniederalkylen oder Niederalkinyloxyniederalkylen ist beispielsweise C₂-C₄-Alkenoxy-C₃-C₄-alkylen, wie 1,3-(3-C₃-C₄-Alkenyloxy)propylen, 1,4-(2-C₃-C₄-Alkenyloxy)butylen, 1,4-(3- C₃-C₄-Alkenyloxy)butylen oder 1,4-(4-C₃-C₄-Alkenyloxy)butylen, bzw. C₂-C₄-Alkinyloxy-C₃-C₄-alkylen, wie 1,3-(2-C₃-C₄-Alkinyloxy)propylen, 1,3-(3-C₃-C₄-Alkinyloxy)propylen, 1,4-(2-C₃-C₄-Alkinyloxy)butylen, 1,4-(3 C₃-C₄-Alkinyloxy)butylen oder 1,4-(4-C₃-C₄-Alkinyloxy)butylen.

C₃-C₄-Alkenyloxy bzw. C₃-C₄-Alkinyloxy hat dabei beispielsweise eine der vorstehenden Bedeutungen und ist insbesondere Allyloxy, Methallyloxy oder Propargyloxy.

Halogen ist beispielsweise Halogen der Atomnummer bis und mit 35, wie Chlor oder Fluor, femer Brom.

Niederalkanoyl ist beispielsweise C₁-C₇-Alkanoyl, insbesondere C₁-C₄-Alkanoyl, wie Formyl, Acetyl, Propionyl, Butyryl oder Isobutyryl, kann aber auch C₅-C₇-Alkanoyl, wie Pivaloyl, sein.

Tautomere von Verbindungen der Formel I werden durch die Strukturformeln Ia oder Ib 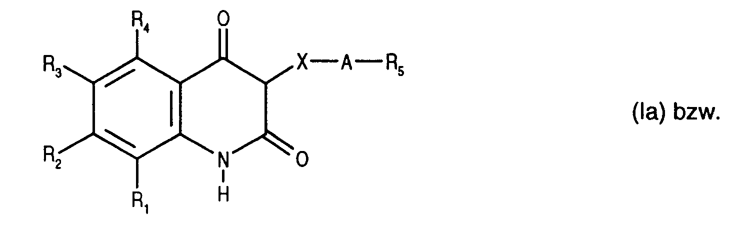 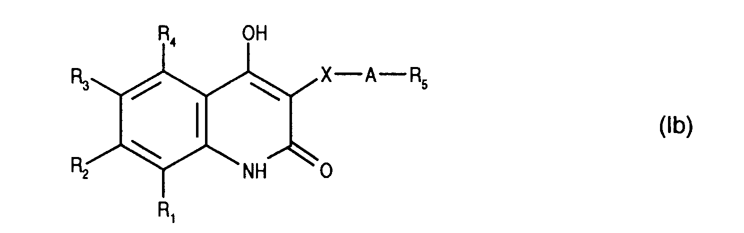
am besten beschrieben.

Verbindungen der Formel I, worin mindestens einer der Reste R₁, R₂, R₃, R₄ und R₅ Carboxy ist, können Salze mit Basen oder, sofem mindestens ein weiterer der Reste R₁, R₂, R₃ und R₄ unsubstituiertes oder aliphatisch substituiertes Amino bedeutet, innere Salze bilden. Verbindungen der Formel 1, worin mindestens einer der Reste R₁, R₂, R₃ und R₄ unsubstituiertes oder aliphatisch substituiertes Amino bedeutet ist, können Säureadditionsalze bilden.

Salze von Verbindungen der Formel I mit Basen sind beispielsweise deren Salze mit pharmazeutisch verwendbaren Basen, wie nicht-toxische, von Metallen der Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, ebenso Ammoniumsalze mit Ammoniak oder organischen Aminen oder quatemären Ammoniumbasen, wie gegebenenfalls C-hydroxylierten aliphatischen Aminen, insbesondere Mono-, Di- oder Triniederalkylaminen, z.B. Methyl-, Ethyl- oder Diethylamin, Mono-, Di- oder Tri-(hydroxyniederalkyl)aminen, wie Ethanol-, Diethanol- oder Triethanolamin, Tris(hydroxymethyl)methylamin oder 2-Hydroxytertiärbutylamin, oder N-(Hydroxyniederalkyl)N,N-diniederalkyl-aminen bzw. N-(Polyhydroxyniederalkyl)-N-niederalkylaminen, wie 2-(Dimethylamino)ethanol oder D-Glucamin, oder quatemären aliphatischen Ammoniumhydroxiden, z.B. Tetrabutylammoniumhydroxid.

Säureadditionssalze von Verbindungen der Formel I sind beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Umfaßt sind sowohl vollständige als auch partielle Salze, d.h. Salze mit 1, 2 oder 3, vorzugsweise 2, Äquivalenten Base pro Mol Säure der Formel I bzw. Salze mit 1, 2 oder 3 Äquivalenten, vorzugsweise 1 Äquivalenten, Säure pro Mol Base der Formel I.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

Die Verbindungen der Formel I weisen wertvolle pharmakologische Eigenschaften auf. Sie besitzen eine selektive nicht-kompetetive antagonistische Wirkung gegenüber N-Methyl-D-asparaginsäure-sensitiven (NMDA-sensitiven) excitatorischen Aminosäurerezeptoren von Warmblütern. Insbesondere vermögen sie an Strychnin-insensitive Glycinmodulatoren des NMDA-Rezeptors zu binden. Das Bindungsvermögen der erfindungsgemäß bereitgestellten Verbindungen und ihrer Salze an Strychnin-insensitive Glycinbindungsstellen des NMDA-Rezeptorkomplexes kann *in vitro* beispielsweise in der Versuchsanordnung nach Baron et al., Eur. J. Pharmacol. , Molec. Pharmacol. Section 206, Seiten 149-154 (1991) und Canton et al., J. Pharm. Pharmacol. 44, Seiten 812-816 (1992) an Rattenkortex- und Rattenhippocampusmembranen ermittelt werden. Dabei wird bestimmt, in welchem Ausmaß [³H]-5,7-Dichlorkynurensäure (³H-DCKA) verdrängt wird, wobei die prozentuale Hemmung und gegebenenfalls bei mehreren Konzentrationen die für eine 50 %-iger Verdrängung erforderliche Konzentration (IC₅₀) bestimmt wird. Die für eine 50 %-ige Verdrängung erforderliche Konzentration (IC₅₀) liegt im Nano- und unteren Millimolbereich, d.h. bei Konzentrationen von etwa 0.07 bis 1.25 µMol.

Auf Grund dieser Eigenschaften sind die Verbindungen der Formel I und ihre pharmazeutisch verwendbaren Salze vorzüglich geeignet zur prophylaktischen und therapeutischen Behandlung von pathologischen Zuständen, die auf Glycin-antagonistische Blockierung von NMDA-sensitiven Rezeptoren ansprechen, beispielsweise von neurodegenerativen Erkrankungen, wie solcher im Gefolge von Schlaganfall, Hypoglykämie, Anoxie oder Hirnlähmungserscheinungen, von ischämischen Erkrankungen, wie der cerebralen Ischämie, der cerebralen Ischämie bei Herzchirurgie oder Herzstillstand, perinataler Aphyxie, epilepstischer Anfälle, chorea Huntington, Alzheimer'scher und Parkinson'scher Erkrankung, amyotroper Lateralsklerose, Rückermark- und Himtrauma sowie Vergiftungserscheinungen durch Neurotoxine oder Suchtmittelmissbrauch, und von ischämischen Erkrankungen des Auges, von Gefäß- und Muskelspasmen, wie von Migräne oder von lokaler oder generaler Spastizität, von Konvulsionen, wie der Epilepsie, und von Angst- und Schmerzzuständen, wie Trigeminusneuralgien.

Die antikonvulsiven Eigenschaften der erfindungsgemäßen Verbindungen können *in vivo* beispielsweise an der Maus anhand ihrer ausgeprägten Schutzwirkung gegenüber durch Metrazol ausgelösten Konvulsionen bestimmt werden, wobei man z.B. das etablierte Mausmodell für Metrazol-induzierte Konvulsionen gemäß Schmutz et al., Naunyn-Schmiedeberg's Arch. Pharmacol. 342, 61-66 (1990) heranziehen kann, in dem Verbindungen der Formel I im Dosisbereich ab etwa 50 mg/kg p.o. und i.p. eine deutliche Schutzwirkung aufweisen.

Die Erfindung betrifft beispielsweise Verbindungen der Formel I, worin die Reste R₁, R₂, R₃ und R₄ unabhängig voneinander Wasserstoff, aliphatische Kohlenwasserstoffreste, gegebenenfalls verethertes Hydroxy, gegebenenfalls verethertes und/oder oxidiertes Mercapto, unsubstituiertes oder aliphatisch substituiertes Amino, Nitro, gegebenenfalls verestertes oder amidiertes Carboxy, Cyano, gegebenenfalls amidiertes Sulfamoyl, Halogen oder Trifluormethyl bedeuten,
X Oxy oder gegebenenfalls oxidiertes Thio darstellt,
A für einen zweiwertigen aliphatischen Rest steht und
R₅ unsubstituiertes oder durch einen aliphatische oder araliphatische Kohlenwasserstoffreste, gegebenenfalls verethertes Hydroxy, gegebenenfalls verethertes und/oder oxidiertes Mercapto, unsubstituiertes oder aliphatisch substituiertes Amino, aliphatisches Acyl, gegebenenfalls verestertes oder amidiertes Carboxy, Cyano, gegebenenfalls amidiertes Sulfamoyl, Halogen und/oder Trifluormethyl substituiertes Aryl oder Hetroaryl, Cyano oder gegebenenfalls verestertes oder amidiertes Carboxy darstellt, und ihre Tautomeren und/oder Salze.

Die Erfindung betrifft in erster Linie Verbindungen der Formel I, worin
die Reste R₁, R₂, R₃ und R₄ unabhängig voneinander Wasserstoff, Niederalkyl, Niederalkenyl, Niederalkinyl, Hydroxy, Niederalkoxy, Niederalkenyloxy, Niederalkinyloxy, Mercapto, Sulfo, Niederalkylthio, Niederalkansulfinyl, Niederalkansulfonyl, Niederalkensulfonyl, Amino, N-Mono- oder N,N-Diniederalkylamino, Nitro, Carboxy, Niederalkoxycarbonyl, unsubstituiertes oder durch Niederalkyl, Niederalkoxy, Hydroxy, Halogen und/oder Trifluormethyl substituiertes Phenylniederalkoxycarbonyl, Carbamoyl, N-Mono- oder N,N-Diniederalkylcarbamoyl, Cyano, Sulfamoyl, N-Mono- oder N,N-Diniederalkylsulfamoyl, Halogen oder Trifluormethyl bedeuten,
X Oxy, Thio, Sulfinyl oder Sulfonyl darstellt,
A für Niederalkylen, Niederalkenylen, Carboxyniederalkylen, Niederalkoxycarbonylniederalkylen, die Hydroxygruppe in höherer als der α-Stellung tragendes Hydroxyniederalkylen, die Niederalkoxygruppe in höherer als der α-Stellung tragendes Niederalkoxyniederalkylen oder die Niederalkenyloxygruppe in höherer als der α-Stellung tragendes Niederalkenyloxyniederalkylen steht und
R₅ einen unsubstituierten oder durch Niederalkyl, Niederalkenyl, Niederalkinyl, Phenylniederalkyl, Hydroxy, Niederalkoxy, Niederalkenyloxy, Niederalkinyloxy, Mercapto, Sulfo, Niederalkylthio, Niederalkansulfinyl, Niederalkansulfonyl, Niederalkensulfonyl Amino, N-Mono- oder N,N-Diniederalkylamino, Niederalkanoyl, Carboxy, Niederalkoxycarbonyl, unsubstituiertes oder durch Niederalkyl, Niederalkoxy, Hydroxy, Halogen und/oder Trifluormethyl substituiertes Phenylniederalkoxycarbonyl, Carbamoyl, N-Mono- oder N,N-Diniederalkylcarbamoyl, Cyano, Sulfamoyl, N-Mono- oder N,N-Diniederalkylsulfamoyl, Halogen und/oder Trifluormethyl mono-, di- oder trisubstituierten monocyclischen oder bicyclischen Arylrest, partiell hydrierten, vorzugsweise über ein gesättigtes C-Atom gebundenen, bicyclischen Arylrest, über ein C-Atom gebundenen, als Heteroatom(e) 1, 2, 3 oder 4 N-Atome oder 1 O- oder S-Atom aufweisenden monocyclischen oder bicyclischen Heteroarylrest oder partiell hydrierten, vorzugsweise über ein gesättigtes C-Atom gebundenen, 1 oder 2 O- oder S-Atome aufweisenden bicyclischen Heteroarylrest, Carboxy, Hydroxymethyl, Phenyloxymethyl, Niederalkoxycarbonyl, Carbamoyl, N-Mono- oder N,N-Diniederalkylcarbamoyl, jeweils unsubstituiertes oder im Phenylteil durch Niederalkyl, Niederalkoxy, Hydroxy, Halogen und/oder Trifluormethyl substituiertes N-Phenylcarbamoyl oder N-Phenyl-N-niederalkyl-carbamoyl oder Cyano darstellt,
und ihre Tautomeren und/oder Salze.

Die Erfindung betrifft vor allem Verbindungen der Formel I, worin
die Reste R₁, R₂, R₃ und R₄ unabhängig voneinander Wasserstoff, C₁-C₄-Alkyl, wie Methoxy, Hydroxy, C₁-C₄-Alkoxy, wie Methoxy, Mercapto, Sulfo, C₁-C₄-Alkylthio, wie Methylthio, C₁-C₄-Alkansulfinyl, wie Methansulfonyl, C₁-C₄-Alkansulfonyl, wie Methansulfonyl, Amino, N-Mono- oder N,N-Diniederalkylamino, wie Dimethyl- oder Diethylamino, Nitro, Carboxy, C₁-C₄-Alkoxycarbonyl, wie Methoxy- oder Ethoxycarbonyl, unsubstituiertes oder durch C₁-C₄-Alkyl, wie Methyl, C₁-C₄-Alkoxy, wie Methoxy, Hydroxy, Halogen der Atomnummer bis und mit 35, wie Chlor, und/oder Trifluormethyl substituiertes Phenyl-C₁-C₄-alkoxycarbonyl, wie Benzyloxycarbonyl oder α-Phenylethoxycarbonyl, Carbamoyl, N-Mono- oder N,N-Di-C₁-C₄-alkylcarbamoyl, wie Methyl- oder Dimethylcarbamoyl, Cyano, Sulfamoyl, N-Mono- oder N,N-Di-C₁-C₄-alkylsulfamoyl, wie Methyl- oder Dimethylsulfamoyl, Halogen der Atomnummer bis und mit 35, wie Chlor, oder Trifluormethyl bedeuten,
X Thio, Sulfinyl, Sulfonyl oder in zweiter Linie Oxy darstellt,
A für jeweils geradkettiges C₂-C₇-Alkylen, insbesondere C₁-C₄-Alkylen, wie Methylen, Ethylen, 1,3-Propylen oder 1,4-Butylen, C₂-C₇-Alkenylen, insbesondere C₂-C₄-Alkylen, wie Vinylen, 1,3-Prop-2-enylen oder 1,4-Butyl-2-enylen, Carboxy-C₁-C₄-alkylen, wie Carboxymethylen, 1- oder 2-Carboxyethylen, 1,3-(1-, 2- oder 3-Carboxy)propylen, 1,4-(1-, 2-, 3-oder 4-Carboxy)butylen, Niederalkoxycarbonylniederalkylen, wie C₁-C₄-Alkoxycarbonylmethylen, 1- oder 2-C₁-C₄-Alkoxycarbonylethylen, 1,3-(1-, 2- oder 3-C₁-C₄-Alkoxycarbonyl)propylen, 1,4-(1-, 2-, 3- oder 4- C₁-C₄-Alkoxycarbonyl)butylen, die Hydroxygruppe in höherer als der α-Stellung tragendes Hydroxyniederalkylen, wie 1,3-(2-Hydroxy)propylen, 1,3-(3-Hydroxy)propylen, 1,4-(2-Hydroxy)butylen, 1,4-(3-C₁-C₄-Hydroxy)butylen, 1,4-(4- Hydroxy)butylen, oder die Niederalkoxygruppe in höherer als der α-Stellung tragendes Niederalkoxyniederalkylen, wie 1,3-(2-C₁-C₄-Alkoxy)propylen, 1,3-(3-C₁-C₄-Alkoxy)propylen, 1,4-(2-C₁-C₄-Alkoxy)butylen, 1,4(3-C₁-C₄-Alkoxy)butylen, 1,4-(4-C₁-C₄-Alkoxy)butylen, steht und
R₅ einen unsubstituierten oder durch C₁-C₄-Alkyl, wie Methyl, Hydroxy, C₁-C₄-Alkoxy, wie Methoxy, Mercapto, Sulfo, C₁-C₄-Alkylthio, wie Methylthio, C₁-C₄-Alkansulfinyl, wie Methansulfinyl, C₁-C₄-Alkansulfonyl, wie Methansulfonyl, Amino, N-Mono- oder N,N-Di-C₁-C₄-alkylamino, wie Dimethyl- oder Diethylamino, Nitro, C₁-C₄-Alkanoyl, wie Acetyl, Carboxy, C₁-C₄-Alkoxycarbonyl, wie Methoxy- oder Ethoxycarbonyl, unsubstituiertes oder durch C₁-C₄-Alkyl, wie Methyl, C₁-C₄-Alkoxy, wie Methoxy, Hydroxy, Halogen der Atomnummer bis und mit 35, wie Chlor, und/oder Trifluormethyl substituiertes Phenyl-C₁-C₄-alkoxycarbonyl, wie Benzyl- oder α-Phenylethoxycarbonyl, Carbamoyl, N-Mono- oder N,N-Di-C₁-C₄-alkylcarbamoyl, wie Methyl- oder Dimethylcarbamoyl, Cyano, Sulfamoyl, N-Mono- oder N,N-Di-C₁-C₄-alkylsulfamoyl, wie Methyl- oder Dimethylsulfamoyl, Halogen der Atomnummer bis und mit 35, wie Chlor, und/oder Trifluormethyl mono-, di- oder trisubstituierten Phenyl-, Naphthyl-Indenyl-, 1,2,3,4-Tetrahydronaphth-1-yl-, 1,2,3,4-Tetrahydronaphth-2-yl-, Indan-1-yl-, Indan2-yl-, Pyrrolyl-, Indolyl-, Furyl-, Benzofuranyl-, Thienyl-, Benzothienyl-, Imidazolyl-, Benzimidazolyl-, Oxazolyl-, Benzoxazoyl-, Thiazoyl-, Benzthiazolyl-, Tetrazolyl-, Pyridyl-, Chinolinyl-, Isochinolinyl-, Pyrimidinyl-, Chinazolinyl-, Pyridazinyl-, Cinnolinyl-, Pyrazinyl-, Chinoxalinyl-, Triazinyl-, Chroman-3-yl-, Chroman-4-yl- oder 1,4-Benzdioxan-2-ylrest, Carboxy, Hydroxymethyl, Phenyloxymethyl, C₁-C₄-Alkoxycarbonyl, wie Methoxy- oder Ethoxycarbonyl, Carbamoyl, N-Mono- oder N,N-Di-C₁-C₄-alkylcarbamoyl, wie Methyl oder Dimethylcarbamoyl, jeweils unsubstituiertes oder im Phenylteil durch C₁-C₄-Alkyl, C₁-C₄-Alkoxy, Hydroxy, Halogen und/oder Trifluormethyl substituiertes N-Phenylcarbamoyl oder N-Phenyl-N-C₁-C₄-alkyl-camamoyl, wie N-Phenyl-N-methyl-carbamoyl, oder Cyano darstellt, beispielsweise solche, worin
die Reste R₁, R₂, R₃ und R₄ unabhängig voneinander Wasserstoff, C₁-C₄-Alkyl, Hydroxy, C₁-C₄-Alkoxy, Mercapto, Sulfo, C₁-C₄-Alkylthio, C₁-C₄-Alkansulfinyl, C₁-C₄-Alkansulfonyl, Amino, N-Mono- oder N,N-Diniederalkylamino, Nitro, Carboxy, C₁-C₄-Alkoxycarbonyl, unsubstituiertes oder durch C₁-C₄-Alkyl, C₁-C₄-Alkoxy, Hydroxy, Halogen der Atomnummer bis und mit 35 und/oder Trifluormethyl substituiertes Phenyl-C₁-C₄-alkoxycarbonyl, Carbamoyl, N-Mono- oder N,N-Di-C₁-C₄-alkylcarbamoyl, Cyano, Sulfamoyl, N-Mono- oder N,N-Di-C₁-C₄-alkylsulfamoyl, Halogen der Atomnummer bis und mit 35 oder Trifluormethyl bedeuten,
X Thio, Sulfinyl, Sulfonyl oder Oxy darstellt,
A für jeweils geradkettiges C₂-C₇-Alkylen, Carboxy-C₁-C₄-alkylen, C₁-C₄-Alkoxycarbonyl-C₁-C₄-alkylen, die Hydroxygruppe in höherer als der α-Stellung tragendes Hydroxyniederalkylen oder die Niederalkoxygruppe in höherer als der α-Stellung Niederalkoxyniederalkylen steht und R₅ unsubstituiertes oder durch C₁-C₄-Alkyl, Hydroxy, C₁-C₄-Alkoxy, Mercapto, Sulfo, C₁-C₄-Alkylthio, C₁-C₄-Alkansulfinyl, C₁-C₄-Alkansulfonyl, Amino, N-Mono- oder N,N-Di-C₁-C₄-alkylamino, Nitro, C₁-C₄-Alkanoyl, Carboxy, C₁-C₄-Alkoxycarbonyl, unsubstituiertes oder durch C₁-C₄-Alkyl, C₁-C₄-Alkoxy, Hydroxy, Halogen der Atomnummer bis und mit 35, wie Chlor, und/oder Trifluormethyl substituiertes Phenyl- C₁-C₄-alkoxycarbonyl, wie Benzyl- oder α-Phenylethoxycarbonyl, Carbamoyl, N-Mono- oder N,N-Di-C₁-C₄-alkylcarbamoyl, wie Methyl- oder Dimethylcarbamoyl, Cyano, Sulfamoyl, N-Mono- oder N,N-Di-C₁-C₄-alkylsulfamoyl, wie Methyl- oder Dimethylsulfamoyl, Halogen der Atomnummer bis und mit 35, wie Chlor, und/oder Trifluormethyl mono-, di- oder trisubstituiertes Phenyl, Carboxy, C₁-C₄-Alkoxycarbonyl, wie Methoxy- oder Ethoxycarbonyl, Carbamoyl, N-Mono- oder N,N-Di-C₁-C₄-alkylcarbamoyl, wie Methyl oder Dimethylcarbamoyl, oder Cyano darstellt, ihre Tautomeren und/oder Salze.

Die Erfindung betrifft insbesondere Verbindungen der Formel I, worin die Reste R₁, R₂, R₃ und R₄ unabhängig voneinander Wasserstoff, C₁-C₄-Alkyl, wie Methyl, C₁-C₄-Alkoxy, wie Methoxy, Mercapto, Sulfo, Nitro, Carboxy, C₁-C₄-Alkoxycarbonyl, wie Methoxy- oder Ethoxycarbonyl, Carbamoyl, N-Mono- oder N,N-Di-C₁-C₄-alkylcarbamoyl, wie Methyl- oder Dimethylcarbamoyl, Cyano, Halogen der Atomnummer bis und mit 35, wie Chlor, oder Trifluormethyl bedeuten, X Thio, Sulfinyl, Sulfonyl oder in zweiter Linie Oxy darstellt, A für jeweils geradkettiges C₂-C₇-Alkylen, insbesondere C₁-C₄-Alkylen, wie Methylen, Ethylen, 1,3-Propylen oder 1,4-Butylen, C₂-C₇-Alkenylen, insbesondere C₂-C₄-Alkylen, wie Vinylen, 1,3-Prop-2-enylen oder 1,4-Butyl-2-enylen, Carboxy-C₂-C₄-alkylen, wie 1- Carboxyethylen, 1,3-(1-Carboxy)propylen oder 1,4-(1-Carboxy)butylen, C₁-C₄-Alkoxycarbonyl-C₂-C₇-alkylen, wie 1-C₁-C₄-Alkoxycarbonylethylen, 1,3-(1-C₁-C₄-Alkoxycarbonyl)propylen oder 1,4-(1- C₁-C₄-Alkoxycarbonyl)butylen, die Hydroxygruppe in höherer als der α-Stellung tragendes Hydroxyniederalkylen, wie 1,3-(2-Hydroxy)propylen, roxy)propylen oder 1,4-(2-Hydroxy)butylen, steht und
R₅ einen unsubstituierten oder durch C₁-C₄-Alkyl, wie Methyl, Hydroxy, C₁-C₄-Alkoxy, wie Methoxy, C₁-C₄-Alkylthio, wie Methylthio, C₁-C₄-Alkansulfinyl, wie Methansulfinyl, C₁-C₄-Alkansulfonyl, wie Methansulfonyl, Nitro, C₁-C₄-Alkanoyl, wie Acetyl, Carboxy, C₁-C₄-Alkoxycarbonyl, wie Methoxy- oder Ethoxycarbonyl, Carbamoyl, Halogen der Atomnummer bis und mit 35, wie Chlor, und/oder Trifluormethyl mono-, di- oder trisubstituierten Phenyl-, Naphthyl-, 1,2,3,4-Tetrahydronaphth-1-yl-, 1,2,3,4-Tetrahydronaphth-2-yl, Thienyl-, Benzimidazolyl-, Oxazolyl-, Benzoxazoyl-, Thiazoyl-, Benzthiazolyl-, Tetrazolyl-, Pyridyl-, Chinolinyl-, Isochinolinyl-, Pyrimidinyl-, Chinazolinyl-, Chroman-3-yl-, Chroman-4-yl- oder 1,4-Benzdioxan-2-ylrest, Carboxy, Hydroxymethyl, Phenyloxymethyl, C₁-C₄-Alkoxycarbonyl, wie Methoxy- oder Ethoxycarbonyl, Carbamoyl, N-Mono- oder N,N-Di-C₁-C₄-alkylcarbamoyl, wie Methyl oder Dimethylcarbamoyl, oder jeweils unsubstituiertes oder im Phenylteil durch C₁-C₄-Alkyl, C₁-C₄-Alkoxy, Hydroxy, Halogen und/oder Trifluormethyl substituiertes N-Phenylcarbamoyl oder N-Phenyl-N-C₁-C₄-alkyl-carbamoyl, wie N-Phenyl-N-methyl-carbamoyl, oder Cyano darstellt,
und ihre Tautomeren und/oder Salze.

Die Erfindung betrifft vorzugsweise Verbindungen der Formel I, worin
R₁ und R₃ Wasserstoff bedeuten,
R₂ Halogen der Atomnummer bis und mit 35 ist und
R₄ Wasserstoff, Halogen der Atomnummer bis und mit 35, wie Chlor, C₁-C₄-Alkyl, wie Ethyl, oder Nitro bedeutet, X Thio darstellt,
A für geradkettiges C₁-C₄-Alkylen, wie Methylen, Ethylen oder 1,3-Propylen, steht und
R₅ einen unsubstituierten oder durch Halogen der Atomnummer bis und mit 35, wie Chlor oder Fluor, Cyano, Carboxy, C₁-C₄-Alkoxycarbonyl, wie Methoxy- oder Ethoxycarbonyl, N-C₁-C₄-Alkylcarbamoyl, wie Methylcarbamoyl, N-Phenyl-N-C₁-C₄-alkylcarbamoyl, wie N-Phenyl-N-methyl-carbamoyl, Hydroxy, C₁-C₄-Alkanoyl, wie Acetyl, und/oder C₁-C₄-Alkyl, wie Methyl mono- oder disubstituierten Phenyl-, Pyrimidinyl-, wie Pyrimidin-5-yl-, Chromanyl-, wie Chroman-2-yl-, 1,4-Benzodioxanyl-, wie 1,4-Benzodioxan-3-yl-, Thienyl-, wie Thien-2-yl- oder Thien-3-yl- oder 1 ,2,3,4-Tetrahydronaphth-2-ylrest, Carboxy, C₁-C₄-Alkoxycarbonyl, wie Methoxy- oder Ethoxycarbonyl, oder N-C₁-C₄-Alkylcarbamoyl, wie Methylcarbamoyl, N-Phenyl-N-C₁-C₄-alkylcarbamoyl, wie N-Phenyl-N-methyl-carbamoyl, darstellt,
beispielsweise solche Verbindungen der Formel I, worin
R₁ und R₃ Wasserstoff bedeuten,
R₂ Halogen der Atomnummer bis und mit 35 ist und
R₄ Wasserstoff oder Halogen der Atomnummer bis und mit 35 bedeutet,
X Thio darstellt,
A für geradkettiges C₁-C₄-Alkylen steht und
R₅ unsubstituiertes oder durch Halogen der Atomnummer bis und mit 35, wie Chlor, Cyano, Carboxy, C₁-C₄-Alkoxycarbonyl, wie Methoxy- oder Ethoxycarbonyl, Hydroxy, C₁-C₄-Alkanoyl, wie Acetyl, und/oder C₁-C₄-Alkyl, wie Methyl mono- oder disubstituiertes Phenyl, Carboxy oder C₁-C₄-Alkoxycarbonyl, wie Methoxy- oder Ethoxycarbonyl, darstellt,
und ihre Tautomeren und/oder Salze.

Die Erfindung betrifft in allererster Linie Verbindungen der Formel I, worin
R₁ und R₃ Wasserstoff bedeutet,
R₂ Halogen der Atomnummer bis und mit 35 ist und R₄ Wasserstoff, Halogen der Atomnummer bis und mit 35 C₁-C₄-Alkyl oder Nitro bedeutet,
X Thio darstellt,
A für geradkettiges C₁-C₄-Alkylen steht und
R₅ unsubstituiertes oder durch Halogen der Atomnummer bis und mit 35, wie Fluor, C₁-C₄-Alkoxy, wie Methoxy, oder Carboxy substituiertes Phenyl, Thienyl, wie Thien-2-yl oder Thien-3-yl, 1,2,3,4-Tetrahydronaphth-2-yl, Pyrimidin-5-yl, Chroman-2-yl, 1,4-Benzodioxan-3-yl, Carboxy oder C₁-C₄-Alkoxycarbonyl, wie Methoxy- oder Ethoxycarbonyl, darstellt, beispielsweise solche, worin
R₁ und R₃ Wasserstoff bedeutet,
R₂ Halogen der Atomnummer bis und mit 35 ist und
R₄ Wasserstoff, C₁-C₄-Alkyl, wie Methyl oder Halogen der Atomnummer bis und mit 35, wie Chlor, bedeutet,
X Thio darstellt,
A für geradkettiges C₁-C₄-Alkylen, wie Methylen, Ethylen oder 1,3-Propylen, steht und
R₅ unsubstituiertes oder durch Halogen der Atomnummer bis und mit 35, wie Chlor, substituiertes Phenyl, Carboxy oder C₁-C₄-Alkoxycarbonyl, wie Methoxy- oder Ethoxycarbonyl, darstellt,
und ihre Tautomeren und/oder Salze.

Die Erfindung betrifft namentlich die in den Beispielen genannten Verbindungen der Formel I und ihre Salze, insbesondere ihre pharmazeutisch verwendbaren Salze.

Das Verfahren zur Herstellung der neuen Verbindungen der Formel I beruht auf an sich bekannten Methoden und ist beispielsweise dadurch gekennzeichnet, daß man
eine Verbindung der Formel II 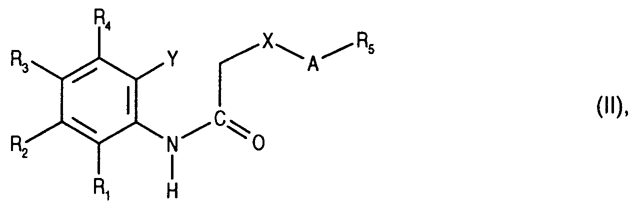
worin
Y eine reaktionsfähige Carboxyfunktion bedeutet,
intramolekular cyclisiert und gewünschtenfalls eine erhaltene Verbindung in eine andere Verbindung der Formel I überführt, ein verfahrensgemäß erhältliches Isomerengemisch in die Komponenten auftrennt und das jeweils bevorzugte Isomere abtrennt und/oder eine verfahrensgemäß erhältliche freie Verbindung in ein Salz oder eine verfahrensgemäß erhältliches Salz in die entsprechende freie Verbindung überführt.

Reaktionsfähige Carboxyfunktionen sind beispielsweise in einer Esterform, Anhydridfomm oder Amidform vorliegende Carboxyfunktionen, wie Niederalkoxycarbonyl, z.B. Methoxy- oder Ethoxycarbonyl, oder gegebenenfalls substituiertes Benzyloxycarbonyl, Niederalkanoyloxycarbonyl, wie Formyloxy- oder Acetoxycarbonyl, Halocarbonyl, z.B. Chlocarbonyl, oder gegebenenfalls substituiertes Carbamoyl, z.B. Carbamoyl, Dimethylcarbamoyl, Piperidinocarbonyl, Thiomorpholinocarbonyl oder Morpholinocarbonyl, ebenso N-Niederalkoxycarbamoyl, wie N-Methoxycarbamoyl, oder N-Niederalkoxy-N-niederalkylcarbamoyl, wie N-Methoxy-N-methyl-carbamoyl.

Die intramolekulare Cyclisierung von Verbindungen der Formel II erfolgt in üblicher Weise, vorzugsweise in Gegenwart einer Metallbase, wie einem gegebenenfalls niederalkylierten oder silylierten Alkalimetallamid, z.B. Natriumamid, oder einem Alkalimetalldiniederalkylamid oder vorzugsweise Alkalimetall-bis-triniederalkylsilyl-amid, insbesondere Lithium-, Natrium- oder Kalium-bis-trimethylsilyl-amid, in einem inerten organischen Lösungsmittel, wie Tetrahydrofuran, Dioxan oder Dimethylformamid, erforderlichenfalls unter Kühlen, z.B. im Temperaturbereich von etwa -25° bis +20°, vorzugsweise zwischen -5° und +5°, und unter mild-saurer Aufarbeitung, beispielsweise Behandeln mit einer verdünnten, wäßrigen Mineralsäure, wie 1 N- bis 3N-Salzsäure.

Die Ausgangsstoffe können nach an sich bekannten Verfahren hergestellt werden, beispielsweise indem man eine Verbindung der Formel III 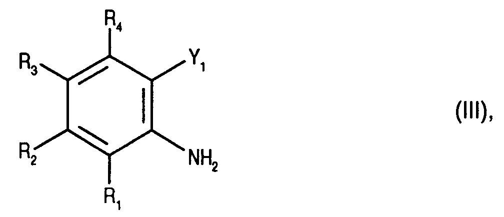
worin Y₁ eine der für Y angegebenen Bedeutungen hat oder für Carboxy steht, in üblicher Weise zunächst mit einem reaktionsfähigen Derivat, wie einem Anhydrid oder Halogenid, einer Halogenessigsäure, z.B. mit Bromessigsäurebromid, und anschließend mit einer Verbindung der Formel IV 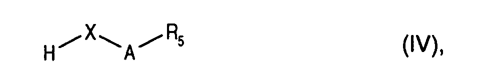
worin X, A und R₅ die angegebenen Bedeutungen haben, kondensiert.

Verfahrensgemäß erhältliche Verbindungen können in üblicher Weise in andere Verbindungen der Formel I überführt werden.

So kann man Verbindungen der Formel I, worin X für Thio oder mindestens einer der Reste R₁, R₂, R₃ und R₄ für verethertes Mercapto steht, in üblicher Weise, beispielsweise durch Behandeln mit m-Chlorperbenzoesäure, zu den entsprechenden Verbindungen der Formel I oxidieren, in denen X für Sulfinyl oder Sulfonyl bzw. mindestens einer der Reste R₁, R₂, R₃ und R₄ für oxidiertes verethertes Mercapto steht.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschließlich ihrer Salze, können auch in Form von Hydraten erhalten werden oder das zur Kristallisation verwendete Lösungsmittel einschließen.

Infolge der engen Beziehung zwischen den neuen Verbindungen in freier Form und in Form ihrer Salze sind vorstehend und nachfolgend unter den freien Verbindungen und ihren Salzen sinn- und zweckgemäß gegebenenfalls auch die entsprechenden Salze bzw. freien Verbindungen zu verstehen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel I enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder D- oder L-(1-Phenyl)ethylamin, 3-Pipecolin, Ephedrin, Amphetamin und ähnliche synthetisch zugängliche Basen, optisch aktive Carbon- oder Sulfonsäuren, wie Chinasäure oder D- oder L-Weinsäure, D- oder L-Ditoluylweinsäure, D- oder L-Äpfelsäure, D- oder L-Mandelsäure, oder D- oder L-Camphersulfonsäure, bzw. optisch aktive Alkohole, wie Bomeol oder D- oder L(1-Phenyl)ethanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemäßen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die Erfindung betrifft gleichfalls pharmazeutische Präparate, die die erfindungsgemäßen Verbindungen oder pharmazeutisch verwendbare Salze derselben als Wirkstoffe enthalten, sowie Verfahren zu ihrer Herstellung.

Bei den erfindungsgemäßen pharmazeutischen Präparaten, welche die erfindungsgemäße Verbindung oder pharmazeutisch verwendbare Salze davon enthalten, handelt es sich um solche zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffes hängt von dem Alter und dem individuellen Zustand sowie von der Applikationsweise ab.

Die neuen pharmazeutischen Präparate enthalten z.B. von etwa 10 % bis etwa 80 %, vorzugsweise von etwa 20 % bis etwa 60 %, des Wirkstoffs. Erfindungsgemäße pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kemen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Traganth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fließ-, Fließregulierund Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaftresistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragéeüberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetisch Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wäßrige Lösungen eines Wirkstoffs in wäßerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet oder wäßrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffes hängt von der Warmblüter-Spezies, dem Alter und dem individuellen Zustand sowie der Applikationsweise ab. Im Normalfall ist für einen etwa 75 kg schweren Patienten bei oraler Applikation eine ungefähre Tagesdosis von etwa 10 mg bis etwa 500 mg zu veranschlagen.

Die nachfolgen Beispiele dienen zur Illustration der Erfindung; Temperaturen sind in Celsiusgraden, Drucke in mbar angegeben.

### Beispiel 1:

2 g (5.3 mM) 4-Chlor-N-(3-phenylpropylthio)acetyl-anthranilsäuremethylester werden in 20 ml Tetrahydrofuran bei 0° vorgelegt. Tropfenweise wird eine 1-molare Tetrahydrofuranlösung von Natrium-bis-trimethylsilylamid zugegeben. Man läßt 20 Minuten bei 0° und 2 Stunden bei Raumtemperatur nachrühren. Das Reaktionsgemisch wird auf 200 ml 2N Salzsäure/Eis gegossen und die entstandene weiße Suspension abfiltriert. Die erhaltenen farblosen Kristalle werden in Ethanol aufgeschlämmt und 2 Stunden verrührt. Man filtriert ab und wäscht mit Ethanol und Diethylether nach. Nach der Trocknung erhält man 7-Chlor-4-hydroxy-3-(3-phenylpropylthio)-2(1 H)-chinolon als farblose Kristalle vom Smp. 184-186°.

Das Ausgangsmaterial kann folgendermaßen hergestellt werden:

10 g (53.9 mMol) 4-Chloranthranilsäuremethylester werden in 400 ml Toluol gelöst und bei Raumtemperatur tropfenweise mit 5.4 ml (62 mMol) Bromacetylbromid versetzt. Man erwärmt auf Rückfluß und läßt 4 Stunden nachrühren. Das Reaktionsgemisch wird auf Raumtemperatur abgekühlt und mit gesättigter Natriumbicarbonatlösung, Wasser und Sole extrahiert. Die wäßrigen Phasen wäscht man mit Toluol nach, vereinigt die organischen Phasen, trocknet über Natriumsulfat, filtriert und dampft ein. Chromatographie an Kieselgel mit Essigester ergibt 4-Chlor-N-(Bromacetyl)anthranilsäuremethylester als farblose Kristalle vom Smp. 95-96°.

4.3 g (28.7 mMol) 3-Phenylpropylthiol werden bei 0° zu einem Gemisch von 1.4 g (31.6 mMol) Natriumhydrid-Dispersion in Öl (55%) und 30 ml Tetrahydrofuran zugetropft. Man läßt 30 Minuten bei 0° nachrühren. Tropfenweise wird eine Lösung von 4-Chlor-N-(Bromacetyl)anthranilsäuremethylester in 30 ml Tetrahydrofuran zugegeben, dann eine Stunde bei 0° und drei Stunden bei Raumtemperatur nachgerührt. Das Reaktionsgemisch versetzt man mit Essigester und extrahiert mit Wasser und Sole. Die wäßrigen Phasen werden mit Essigester nachgewaschen, die organischen Phasen vereinigt, über Natriumsulfat getrocknet, filtriert und eingedampft. Chromatographie an Kieselgel mit Hexan/Essigester (4:1) ergibt 4-Chlor-N-(3-phenylpropylthio)acetylanth ranilsäu remethylester als gelbes Öl.

### Beispiel 2:

Durch Cyclisierung von 4-Chlor-6-ethyl-N-(3-phenylpropylthioacetyl)-anthranilsäuremethylester mittels Natrium-N,N-bis(trimethylsilyl)amid in Tetrahydrofuran erhält man 7-Chlor-5-ethyl-4-hydroxy-3-(3-phenylpropylthio)-2(1 H)-chinolon vom Smp. 183-185°.

Das Ausgangsmaterial kann z.B. durch Umsetzung von 6-Ethyl-4-chlor N-bromacetyl-anthranilsäuremethylester mit 3-Phenylpropylthiol in Gegenwart von Natriumhydrid hergestellt werden.

### Beispiel 3:

Durch Cyclisierung von 4-Chlor-N-(2-phenethylthioacetyl)-anthranilsäuremethylester mittels Natrium-N,N-bis(trimethylsilyl)amid in Tetrahydrofuran erhält man 7-Chlor-4-hydroxy-3-(2-phenethylhio)-2(1 H)-chinolon vom Smp. 213-215°.

### Beispiel 4:

Durch Cyclisierung von 4-Chlor-6-(hex-1-enyl)-N-(3-phenylpropylthioacetyl)anthranilsäuremethylester mittels Natrium-N,N-bis(trimethylsilyl)amid in Tetrahydrofuran erhält man 7-Chlor-5-(hex-1-enyl)-4-hydroxy-3-(3-phenylpropylthio)-2(1H)-chinolon vom Smp. 145-148°.

Das Ausgangsmaterial kann z.B. durch Umsetzung von 4-Chlor-6-jod-N-bromacetylanthranilsäuremethylester mit Hexen-1-yl-tributylstannan in Gegenwart von Triphenylphospinpalladiumchlorid und Lithiumchlorid in Dimethylformamid und anschließende Umsetzung des so erhältlichen 4-Chlor-6-(hex-1-enyl)-N-bromacetyl-anthranilsäuremethylester mit 3-Phenylpropylthiol in Gegenwart von Natriumhydrid hergestellt werden.

### Beispiel 5:

Durch Cyclisierung von 4,6-Dichlor-N-[3-(4-methoxycarbonylphenyl)propylthioacetyl]-anthranilsäuremethylester mittels Natrium-N,N-bis(trimethylsilyl)amid in Tetrahydrofuran erhält man 5,7-Dichlor-4-hydroxy-3-[3-(4-methoxycarbonylphenyl)propylthio]--2(1H)-chinolon und durch Hydrolyse desselben mittels 2N Natronlauge 5,7-Dichlor-4-hydroxy-3-[3-(4-carboxyphenyl)propylthio]-2(1H)-chinolon vom Smp. 275-277°.

Das Ausgangsmaterial kann z.B. durch Umsetzung von 4,6-Dichlor N-bromacetyl-anthranilsäuremethylester mit 3-(4-Methoxycarbonylphenyl)propylthiol (erhältlich aus dem Bromid) in Gegenwart von Natriumhydrid hergestellt werden.

### Beispiel 6:

Durch Cyclisierung von 4,6-Dichlor-N-[3-(3-methoxycarbonylphenyl)propylthioacetyl]-anthranilsäuremethylester mittels Natrium-N,N-bis(trimethylsilyl)amid in Tetrahydrofuran erhält man 5,7-Dichlor-4-hydroxy-3-[3-(3-methoxycarbonylphenyl)propylthio]--2(1H)-chinolon und durch Hydrolyse desselben mittels 2N Natronlauge 5,7-Dichlor-4-hydroxy-3-[3-(3-carboxyphenyl)propylthio]-2(1H)-chinolon vom Smp. 269-272°.

Das Ausgangsmaterial kann z.B. durch Umsetzung von 4,6-Dichlor N-bromacetyl-anthranilsäuremethylester mit 3-(3-Methoxycarbonylphenyl)propylthiol (erhältlich aus dem Bromid) in Gegenwart von Natriumhydrid hergestellt werden.

### Beispiel 7:

2.0 g (6.03 mMol) 4-Chlor-N-(Methoxycarbonylmethylthioacetyl)-anthranilsäuremethylester werden in 20 ml Tetrahydrofuran bei 0° vorgelegt und 290 mg (6.63 mMol) Natriumhydrid-Dispersion in Öl (55%) zugegeben. Man läßt 15 Minuten bei 0° und 3 Stunden bei Raumtemperatur nachrühren. Dann wird das Reaktionsgemisch auf Eiswasser gegossen, mit 1N Salzsäure auf pH 3 gestellt und mit Essigester extrahiert. Die organischen Phasen werden vereinigt, mit Natriumsulfat getrocknet, abfiltriert und eingeengt. Man erhält 7-Chlor-4-hydroxy-3-(methoxycarbonylmethylthio)-2(1H)-chinolon, welches gemäß Beispiel 8 als Säure charakterisiert werden kann..

Das Ausgangsmaterial kann folgendermaßen hergestellt werden:

21.24 g (0.114 Mol) 4-Chloranthranilsäuremethylester und 19.1 ml (0.137 Mol) Triethylamin werden in 180 ml Methylenchlorid bei 0° vorgelegt. Tropfenweise wird eine Lösung von 20.9 g (0.114 Mol) 2-(Chloroformylmethylthio)-essigsäure-methylester in 20 ml Methylenchlorid zugegeben und 15 Minuten bei 0° und 3 Stunden bei Raumtemperatur nachgerührt. Man extrahiert das Reaktionsgemisch mit Wasser und Sole und wäscht die wäßrigen Phasen mit Methylenchlorid. Die organischen Phasen werden vereinigt, mit Natriumsulfat getrocknet, filtriert und eingeengt. Chromatographie des Rohprodukts an Kieselgel mit Hexan/Essigester (4:1) ergibt noch unreinen 4-Chlor-N-(Methoxycarbonylmethylthioacetyl) anthranilsäuremethylester als gelbliche Kristalle.

### Beispiel 8:

Das gemäß Beispiel 7 erhaltene 7-Chlor-4-hydroxy-3-(methoxycarbonylmethylthio)-2(1H)-chinolon wird in 2N Natronlauge gelöst und 2 Stunden bei Raumtemperatur stehen gelassen. Man stellt das Reaktionsgemisch mit 4N Salzsäure auf pH 2 und filtriert die erhaltene Suspension ab. Die farblosen Kristalle werden am Hochvakuum bei 60° getrocknet. Man erhält 7-Chlor-4-hydroxy-3-(carboxymethylthio)-2(1 H)-chinolon als reine Kristalle vom Smp. 288° (Zers.).

### Beispiel 9:

2.0 g (6.03 mMol) 4-Chlor-N-(methoxycarbonylthio)acetyl-anthranilsäuremethylester werden in 20 ml Tetrahydrofuran bei 0° vorgelegt und 290 mg (6.63 mMol) Natriumhydrid-Dispersion in Öl (55%) zugegeben. Man läßt 15 Minuten bei 0° und 3 Stunden bei Raumtemperatur nachrühren. Dann wird das Reaktionsgemisch auf Eiswasser gegossen, mit 1N Salzsäure auf pH 3 gestellt und mit Essigester extrahiert. Die organischen Phasen werden vereinigt, mit Natriumsulfat getrocknet, abfiltriert und eingeengt. Das Zwischenprodukt wird in 2N Natronlauge gelöst und 2 Stunden bei Raumtemperatur stehen gelassen. Man stellt das Reaktionsgemisch mit 4N Salzsäure auf pH 2 und filtriert die erhaltene Suspension ab. Die farblosen Kristalle werden am Hochvakuum bei 60° getrocknet. Man erhält 7-Chlor-4-hydroxy-3-(carboxymethylthio)-2(1 H)-chinolon als reine Kristalle vom Smp. 288° (Zers.).

Das Ausgangsmaterial kann beispielsweise wie folgt hergestellt werden:

21.24 g (0.114 Mol) 4-Chloranthranilsäuremethylester und 19.1 ml (0.137 Mol) Triethylamin werden in 180 ml Methylenchlorid bei 0° vorgelegt. Tropfenweise wird eine Lösung von 20.9 g (0.114 Mol) 2-(Chloroformylmethylthio)-essigsäuremethylester (T. Terasawa and T. Okada, J. Org. Chem., 42,1163 (1977)) in 20 ml Methylenchlorid zugegeben und 15 Minuten bei 0° und 3 Stunden bei Raumtemperatur nachgerührt. Man extrahiert das Reaktionsgemisch mit Wasser und Sole und wäscht die wäßrigen Phasen mit Methylenchlorid. Die organischen Phasen werden vereinigt, mit Natriumsulfat getrocknet, filtriert und eingeengt. Chromatographie des Rohprodukts an Kieselgel mit Hexan/Essigester (4:1) ergibt noch unreinen 4-Chlor-N-(methoxycarbonylmethylthio)acetylanthranilsäu remethylester als gelbliche Kristalle.

### Beispiel 10:

Durch Cyclisierung von 4,6-Dichlor-N-(1-methoxycarbonylmethylthioacetyl)anthranilsäuremethylester mittels Natrium-N,N-bis(trimethylsilyl)amid in Tetrahydrofuran und anschießende Hydrolyse mittels 2N Natronlauge erhält man 5,7-Dichlor-4-hydroxy-3-(carboxymethylthio)-2(1H)-chinolon vom Smp > 300°.

### Beispiel 11:

2.0 g (5.6 mMol) 4-Chlor-N-(3-methoxycarbonylpropylthio)acetyl-anthranilsäuremethylester werden in 20 ml Tetrahydrofuran bei 0° vorgelegt und tropfenweise 16.8 ml einer 1 M Lösung von Natrium-bis-trimethylsilylamid zugegeben. Man läßt 30 Minuten bei 0° nachrühren und gießt das Reaktionsgemisch auf 2 N Salzsäure / Eis. Die entstandene weiße Suspension wird abgenutscht und die erhaltenen farblosen Kristalle am Hochvakuum bei 60° getrocknet. Das getrocknete Produkt wird in Essigester aufgeschlämmt und 30 Minuten verrührt. Man filtriert die Suspension ab, trocknet die farblosen Kristalle am Hochvakuum bei 70° und erhält 7-Chlor-4-hydroxy-3-(3-methoxycarbonylpropylthio)2(1H)-chinolon als farblose Kristalle vom Smp.183-85° (Zers.).

Das Ausgangsmaterial kann beispielsweise wie folgt hergestellt werden:

3.5 g (26.1 mM) 4-Thiobuttersäuremethylester werden zu einer Suspension von 1.4 g (31.3 mMol) Natriumhydrid-Dispersion in Öl (55 %) und 20 ml Tetrahydrofuran zugetropft. Man läßt 20 Minuten bei Raumtemperatur nachrühren, gibt dann tropfenweise eine Lösung von 6.3 g N-Bromacetyl-4-chlor-anthranilsäuremethylester in 60 ml Tetrahydrofuran zu und rührt weitere zwei Stunden bei Raumtemperatur. Das Reaktionsgemisch wird mit Essigester versetzt und mit Wasser und Sole extrahiert. Die wäßrigen Phasen werden mit Essigester gewaschen, die organischen Phasen vereinigt, mit Natriumsulfat getrocknet, filtriert und eingeengt. Chromatographie des Rohproduktes an Kieselgel mit Hexan/Essigester (3:1) ergibt unreinen 4-Chlor-N-(3-methoxycarbonylpropylthio)acetylanthranilsäuremethylester als farblosen Kristalle vom Smp. 57 - 59°.

### Beispiel 12:

895 mg (2.73 mMol) 7-Chlor-4-hydroxy-3-(3-methoxycarbonylpropylthio)-2(1H)-chinolon werden in 10 ml 2N Natronlauge gelöst und eine Stunde bei Raumtemperatur verrührt. Das Reaktionsgemisch wird mit konzentrierter Salzsäure auf pH 2 gestellt, die entstandene weiße Suspension abfiltriert und mit Wasser nachgewaschen. Die erhaltenen farblosen Kristalle trocknet man am Hochvakuum bei 70° und erhält 3-(3-Carboxypropylthio)-7-chlor-4-hydroxy-2(1H)-chinolon als farblose Kristalle vom Smp. 239-41° (Zers.).

### Beispiel 13:

6.3 g (16.12 mMol) 4-Chlor-N-(4-phenylbutylthio)acetyl-anthranilsäuremethylester werden in 60 ml Tetrahydrofuran bei 0° vorgelegt und tropfenweise 48.3 ml einer 1-molaren Lösung von Natrium-bis-trimethylsilylamid zugegeben. Man läßt 30 Minuten bei Raumtemperatur nachrühren. Das Reaktionsgemisch wird auf 200 ml 2N Salzsäure/Eis gegossen, die entstandene weiße Suspension abgenutscht und die farblosen Kristalle im Hochvakuum bei 60° getrocknet. Man erhält 7-Chlor-3-(4-phenylbutylthio)-4-hydroxy-2(1H)chinolon als farblose Kristalle vom Smp.176°.

Das Ausgangsmaterial kann beispielsweise wie folgt hergestellt werden:

3.57 g (47.1 mMol) Thiohamstoff werden in 11 ml Ethanol zum Rückfluß erwärmt und tropfenweise eine Lösung von 10 g (46.9 mMol) 4-Phenylbutylbromid in 23 ml Ethanol zugetropft. Man hält das Reaktionsgemisch 4 Stunden am Rückfluß, kühlt dann ab und dampft ein. Das Rohprodukt wird aus Tetrahydrofuran und Diethylether umkristallisiert. Man erhält S-(4-Phenylbutyl)isothiuroniumbromid als nicht ganz reine Kristalle.

11 g (44.1 mMol) S-(4-Phenylbutyl)isothiuroniumbromid werden in 68 ml Wasser vorgelegt und eine warme Lösung von 3.71 g (92.7 mMol) Natriumhydroxid in 14 ml Wasser zugetropft. Das Reaktionsgemisch wird mit Kochsalz gesättigt und mit Ether extrahiert. Die organischen Phasen werden vereinigt, mit Natriumsulfat getrocknet, filtriert und eingeengt. Man erhält 4-Phenylbutylthiol als farblose Flüssigkeit, die roh weiterumgesetzt wird.

1.85 g (42.5 mMol) Natriumhydrid-Dispersion in Öl (55 %) werden in 45 ml Tetrahydrofuran bei 0° vorgelegt und tropfenweise 6.4 g (38.6 mMol) 4-Phenylbutylthiol zugegeben. Man läßt 30 Minuten bei 0° nachrühren und tropft dann eine Lösung von 10.76 g (35.1 mMol) N-Bromacetyl-4-chlor-anthranilsäuremethylester in 45 ml Tetrahydrofuran zu. Man rührt eine Stunde bei 0° und 1.5 Stunden bei Raumtemperatur nach. Das Reaktionsgemisch wird mit Essigester versetzt, mit Wasser und Sole extrahiert und die wäßrigen Phasen mit Essigester gewaschen. Die organischen Phasen werden vereinigt, mit Natriumsulfat getrocknet, filtriert und eingeengt. Chromatographie des Rohproduktes an Kieselgel ergibt 4-Chlor-N-(4-phenylbutylthio)acetylanthranilsäu remethylester als gelbes Öl.

### Beispiel 14:

0.3 g (6.9 mMol) Natriumhydrid-Dispersion in Öl (55 %) werden in 15 ml Tetrahydrofuran bei 0° vorgelegt. Tropfenweise wird eine Lösung von 2.2 g (6.3 mMol) N-(Benzylthio)acetyl-4-chlor-anthranilsäuremethylester in 5 ml Tetrahydrofuran zugegeben. Man läßt 24 Stunden bei Raumtemperatur nachrühren. Das Reaktionsgemisch wird auf Eiswasser gegossen, mit 2 N Salzsäure auf pH 3 gestellt und mit Essigester extrahiert. Die organischen Phasen werden mit Wasser und Sole gewaschen, vereinigt, mit Natriumsulfat getrocknet, abgenutscht und eingeengt. Kristallisation des Rohproduktes aus Essigester/Hexan ergibt nach Trocknung 7-Chlor-3-(Benzylthio)-4-hydroxy-2(1H)-chinolon als farblose Kristalle vom Smp. 228-230°.

Das Ausgangsmaterial kann beispielsweise wie folgt hergestellt werden:

5.1 g (27.4 mMol) 4-Chlor-anthranilsäuremethylester und 5.73 ml (41.1 mMol) Triethylamin werden in 40 ml Methylenchlorid vorgelegt und tropfenweise eine Lösung von 5.5 g (27.4 mMol) Benzylthioacetylchlorid in 5 ml Methylenchlorid zugegeben. Man läßt eine Stunde bei Raumtemperatur nachrühren. Das Reaktionsgemisch wird mit Wasser und Sole extrahiert. Die wäßrigen Phasen wäscht man mit Methylenchlorid nach. Die organischen Phasen werden vereinigt, mit Natriumsulfat getrocknet, filtriert und eingeengt. Chromatographie des Rohproduktes an Kieselgel mit Hexan/Essigester (6:1) ergibt N-(Benzylthio)acetyl-4-chloranthranilsäuremethylester als gelbes Öl.

### Beispiel 15:

1.0 g (2.73 mMol) N-(Benzylsulfinyl)acetyl-4-chlor-anthranilsäuremethylester werden in 10 ml Tetrahydrofuran bei 0° vorgelegt und mit 0.13 g (3 mMol) Natriumhydrid-Dispersion in Öl (55%) versetzt. Man läßt 15 Minuten bei 0° und 2 Stunden bei Raumtemperatur nachrühren. Das Reaktionsgemisch wird mit Wasser versetzt, mit 1N Salzsäure auf pH 2-3 gestellt und mit Essigester extrahiert. Die organischen Phasen werden mit Wasser und Sole gewaschen, vereinigt, mit Natriumsulfat getrocknet, filtriert und eingeengt. Kristallisation des Rohproduktes aus Essigester/Petrolether ergibt 7-Chlor-3-(benzylsulfinyl)-4-hydroxy-2-(1 H)-chinolon als farblose Kristalle vom Smp. 234° (Zers.).

Das Ausgangsmaterial kann beispielsweise wie folgt hergestellt werden:

2 g (5.7 mMol) N-(Benzylthio)acetyl-4-chlor-anthranilsäuremethylester und 3.66 g (17.1 mMol) Natriummetaperjodat werden in 12 ml Wasser und 48 ml Aceton gelöst und 6 h am Rückfluß gerührt. Man läßt das Reaktionsgemisch abkühlen, filtriert und dampft ein. Chromatographie des Rohproduktes an Kieselgel mit Hexan/Essigester ergibt N-(Benzylsulfinyl)acetyl-4-chlor-anthranilsäuremethylester als gelbes, mit Kristallen durchsetztes Öl.

### Beispiel 16:

In analoger Weise wie in den Beispielen 1 bis 15 beschrieben kann man durch Cyclisierung von 4-Chlor-N-[1-(1-methoxycarbonyl-3-phenyl-propyl)thioacetyl]-anthranilsäuremethylester 7-Chlor-4-hydroxy-3-(1-methoxycarbonyl-3-phenyl-propylthio)-2(1H)--chinolon und durch anschließende Hydrolyse desselben 7-Chlor-4-hydroxy-3-(1-carboxy-3-phenyl-propylthio)-2(1 H)-chinolon vom Smp.262-266° herstellen.

Das Ausgangsmaterial kann z.B. folgendermaßen hergestellt werden:
2.3 g (53.2 mMol) Natriumhydrid als Suspension in Mineralöl werden bei 0° in 30 ml Tetrahydrofuran vorgelegt. Dann werden bei 0 bis 10° 6.6 g (44.3 mMol) Thioglykolsäuretertiärbutylester zugetropft. Es bildet sich eine dickflüssige Suspension, die mit 50 ml Tetrahydrofuran verdünnt wird. Man läßt 20 Minuten bei 0° nachrühren und tropft dann 12 g (44.3 mMol) 2-Brom-4-phenyl-buttersäuremethylester hinzu. Man läßt 2 Stunden bei Raumtemperatur nachrühren, nimmt in 100 ml Essigsäureethylester auf und schüttelt nacheinander mit 50 ml Wasser und 50 ml gesättigter Kochsalzlösung aus. Die Wasserphasen werden vereinigt und mit 25 ml Essigsäureethylester ausgeschüttelt. Die organischen Phasen werden vereinigt, über Natriumsulfat getrocknet, abfiltriert und eingedampft. Chromatographie an Kieselgel mit Hexan/Essigsäureethylester (20:1) als Laufmittel ergibt 5.7 g (38% d.Th.) 2-Tertiärbutyloxycarbonylmethylthio-4-phenyl-buttersäu reethylester als Öl.

5.6 g (16.5 mMol) 2-Tertiärbutyloxycarbonylmethylthio-4-phenyl-buttersäureethylester werden bei Raumtemperatur in 60 ml Dichlormethan vorgelegt. Man gibt 3.8 ml (49.6 mMol) Trifluoressigsäure hinzu, läßt 16 Stunden bei Raumtemperatur rühren, gibt weitere 3.8 ml Trifluoressigsäure hinzu, läßt 24 Stunden bei Raumtemperatur nachrühren und wäscht nacheinander mit je 25 ml Wasser und 25 ml gesättigter Kochsalzlösung. Die wäßrigen Phasen werden mit 2550 ml Dichlormethan nachgewaschen. Die vereinigten organischen Phasen werden über Natriumsulfat getrocknet, abfiltriert und eingedampft. Man erhält 4.86 g (100% d.Th.) rohen 2-Carboxymethylthio-4-phenyl-buttersäureethylester als braunes Öl.

4.6 g (16.3 mMol) 2-Carboxymethylthio-4-phenyl-buttersäureethylester und 1.8 ml (16.3 mMol) 4-Methylmorpholin werden unter Stickstoff bei 0° in 100 ml Dichlormethan vorgelegt. Dann werden 2.1 ml (16.3 mMol) Chlorameisensäurebutylester zugetropft. Man läßt 30 Minuten bei 0° rühren, gibt 3 g (16.3 mMol) 4-Chloranthanilsäuremethylester hinzu, läßt 16 Stunden nachrühren und dampft ein. Chromatographie an Kieselgel mit Hexan/Essigsäureethylester (7:1) als Laufmittel ergibt 1.47 g (20% d.Th.) 4-Chlor-N-[1-(1-methoxycarbonyl-3-phenyl-propyl)thioacetyl]-anth ranilsäu remethylester als gelbliches Öl.

### Beispiel 17:

Durch Cyclisierung von 4-Chlor-N-[1-(2-acetoxy-3-phenyl-propyl)thioacetyl]anthranilsäuremethylester mittels Natrium-N,N-bis(trimethylsilyl)amid in Tetrahydrofuran und anschießende Hydrolyse mittels 2N Natronlauge erhält man 7-Chlor-4-hydroxy-3-(2-hydroxy-3-phenyl-propylthio)-2(1 H)-chinolon vom Smp. 158-160°.

Das Ausgangsmaterial kann z.B. folgendermaßen hergestellt werden:

8 g (37.2 mMol) 2-Hydroxy-3-phenyl-propylbromid werden bei Raumtemperatur in 80 ml Dichlormethan gelöst, unter Rühren tropfenweise mit 2.64 ml (37.2 mMol) Acetylchlorid und nach 3 Stunden mit weiteren 2.64 ml (37.2 mMol) Acetylchlorid versetzt, , 4 Stunden bei 60° und 16 Stunden bei Raumtemperatur nachgerührt und mit je 25 ml Wasser und gesättigter Kochsalzlösung gewaschen. Die wäßrigen Phasen werden vereinigt und mit 30 ml Dichlormethan gewaschen. Die vereinigten organischen Phasen werden über Natriumsulfat getrocknet und eingedampft. Chromatographie an Kieselgel mit Hexan/Essigsäureethylester (19:1) als Laufmittel ergibt 8.03 g (83% d.Th.) 2-Acetoxy-3-phenyl-propylbromid als farblose Flüssigkeit.

Aus 2-Acetoxy-3-phenyl-propylbromid erhält man durch Kondensation mit Thioglykolsäuretertiärbutylester in Gegenwart von Natriumhydrid 2-Acetoxy-3-phenyl-propylthioessigsäuretertiärbutylester.

Durch Behandlung von 2-Acetoxy-3-phenyl-propylthioessigsäuretertiärbutylester mit Trifluoressigsäure erhält man 2-Acetoxy-3-phenyl-propylthioessigsäure und aus dieser durch Behandlung mit Phosgen 2-Acetoxy-3-phenyl-propylthioacetylchlorid.

Aus 2-Acetoxy-3-phenyl-propylthioacetylchlorid erhält man durch Umsetzung mit 4-Chloranthranilsäuremethylester 4-Chlor-N-[1-(2-acetoxy-3-phenyl-propyl)thioacetyl]-anthranilsäuremethylester.

### Beispiel 18:

Durch Cyclisierung von 4-Chlor-N-[1-(2-acetoxyethyl)thioacetyl]-anthranilsäuremethylester mittels Natrium-N,N-bis(trimethylsilyl)amid in Tetrahydrofuran und anschießende Hydrolyse mittels 2N Natronlauge erhält man 7-Chlor-4-hydroxy-3-(2-hydroxyethylthio)-2(1H)-chinolon vom Smp. 233-236°.

Das Ausgangsmaterial kann z.B. durch Umsetzung von 4-Chlor N-bromacetyl-anthranilsäuremethylester mit 2-Acetoxyethylthiol in Gegenwart von Natriumhydrid hergestellt werden.

### Beispiel 19:

In analoger Weise wie in Beispiel 15 beschrieben kann man durch Oxidation von N-(Benzylthio)acetyl-4-chlor-anthranilsäuremethylester mit m-Chlorperbenzoesäure in Dichlormethan und Cyclisierung des so erhältlichen N-(Benzylsulfonyl)acetyl-4-chlor-anthranilsäuremethylester 7-Chlor-3-(benzylsulfonyl)-4-hydroxy-2-(1H)-chinolon herstellen.

### Beispiel 20:

In analoger Weise wie in Beispiel 7 beschrieben kann man durch Cyclisierung von N-(Methoxycarbonylmethansulfonyl)acetyl-4-chlor-anthranilsäuremethylester 7-Chlor-3-(methoxycarbonylmethansulfonyl)-4-hydroxy-2-(1H)-chinolon und aus diesem durch Hydrolyse mittels Natronlauge 7-Chlor-3-(carboxymethansulfonyl)-4-hydroxy-2-(1H)-chinolon herstellen.

Das Ausgangsmaterial erhält man beispielsweise durch Oxidation von N-(Methoxycarbonylmethylthio)acetyl-4-chlor-anthranilsäuremethylester mit m-Chlorperbenzoesäure in Dichlormethan.

### Beispiel 21:

In analoger Weise wie in Beispiel 1 beschrieben kann man durch Cyclisierung von 4,6-Dichlor-N-[3-(4-methoxyphenyl)propylthio)acetyl-anthranilsäuremethylester 5,7-Dichlor-3-[3-(4-methoxyphenyl)propylthio)-4-hydroxy-2-(1H)-chinolon herstellen.

### Beispiel 22:

In analoger Weise wie in Beispiel 14 beschrieben kann man durch Cyclisierung von 4,6-Dichlor-N-[3-(2-fluorphenyl)propylthio)acetyl-anthranilsäuremethylester 5,7-Dichlor-3-[3-(2-fluorphenyl)propylthio)-4-hydroxy-2-(1H)-chinolon herstellen.

### Beispiel 23:

In analoger Weise wie in Beispiel 1 beschrieben kann man durch Cyclisierung von 4,6-Dichlor-N-(benzylthio)acetyl-4-chlor-anthranilsäuremethylester 5,7-Dichlor-3-(benzylthio)-4-hydroxy-2-(1 H)-chinolon herstellen.

### Beispiel 24:

In analoger Weise wie in Beispiel 14 beschrieben kann man durch Cyclisierung von 4,6-Dichlor-N-[3-(4-fluorphenyl)propylthio)acetyl-anthranilsäuremethylester 5,7-Dichlor-3-[3-(4-fluorphenyl)propylthio)-4-hydroxy-2-(1H)-chinolon herstellen.

### Beispiel 25:

0.55 g (1.3 mM) 4-Chlor-6-nitro-N-(3-phenylpropylthio)acetyl-anthranilsäuremethylester werden in 6 ml Tetrahydrofuran bei 0° unter Stickstoff vorgelegt. Tropfenweise gibt man 2.6 ml einer 1-molaren Tetrahydrofuranlösung von Natrium-bis-trimethylsilylamid hinzu. Man läßt 1 Stunde bei 0° nachrühren. Das Reaktionsgemisch wird auf 50 ml 2N Salzsäure/Eis gegossen und mit Essigsäureethylester extrahiert. Die organische Phase wird über Natriumsulfat getrocknet, abfiltriert und eingedampft. Der Rückstand wird in wenig Essigsäureethylester aufgenoommen und bis zur beginnenden Fällung mit Hexan versetzt. Der gebildete Niederschlag wird abgenutscht und getrocknet. Man erhält 374 mg (73% d.Th.) 7-Chlor-5-nitro-4-hydroxy-3-(3-phenylpropylthio)-2(1H)-chinolon als rote Kristalle vom Smp.177-184°.

Das Ausgangsmaterial kann folgendermaßen hergestellt werden:

14.2 g (82.3 mMol) 3-Chlor-5-nitro-anilin werden unter Stickstoff bei -65° in 235 ml Dichlormethan gelöst und tropfenweise mit einer Lösung von 9.8 ml (82.3 mMol) Tertiärbutylhypochlorit in 30 ml Dichlormethan versetzt. Man läßt 10 Minuten bei -65° nachrühren und tropft dann eine Lösung von 10.6 ml (82.3 mMol) Methylthioessigsäureethylester in 30 ml Dichlormethan hinzu. Man läßt nochmals 1 Stunde bei -65° nachrühren und tropft dann eine Lösung von 11.5 ml (82.3 mMol) Triethylamin ein. Man läßt auf Raumtemperatur erwärmen, wäscht mit wenig Wasser, trocknet über Natriumsulfat, filtriert und dampft ein. Der Eindampfrückstand wird in 250 ml Diethylether aufgenommen, mit 30 ml 2N Salzsäure versetzt und 16 Stunden bei Raumtemperatur gerührt. Der rote Niederschlag wird abfiltriert. mit Diethylether nachgewaschen und getrocknet. Man erhält 8.48 g (40% d.Th.) 6-Chlor-3-methylthio-4-nitrochinolin-2-on vom Smp. 213° (Zers.)

8 g (31 mMol) 6-Chlor-3-methylthio-4-nitrochinolin-2-on und 15.8 g (93 mMol) Kupfer-II-chlorid-dihydrat werden in 215 ml Aceton und 24 ml Wasser 1.5 Stunden unter Rühren zum Rückfluß erhitzt. Man gießt in 600 ml Toluol, filtriert ab, wäscht mit wenig Wasser nach trocknet über Natriumsulfat, filtriert und dampft zur Trockne ein. Der Rückstand wird in wenig Essigsäureethylester aufgeschlämmt, 20 Minuten gerührt, bis zur beginnenden Niederschlagsbildung mit Hexan versetzt, abgesaugt und getrocknet. Man erhält 4.28 g (60.9% d.Th.) 6-Chlor-4-nitro-indolin-2,3-dion als gelbe Kristalle vom Smp. 227-231°.

3.8 g (16.7 mMol) .) 6-Chlor-4-nitro-indolin-2,3-dion in 60 ml Natronlauge werden bei Raumtemperatur tropfenweise mit 4 ml einer 30%-igen Wasserstoffperoxidlösung versetzt. Man läßt 2 Stunden bei Raumtemperatur rühren, säuert mit 4N Salzsäure auf pH 2 bis 3 an, saugt ab und trocknet. Man erhält 3.18 g (87.8% d.Th.) 4-Chlor-6-nitro-anthranilsäure vom Smp. 202-205°.

3.25 g (15 mMol) .) 4-Chlor-6-nitro-anthranilsäure und 9,73 ml einer 40%-igen wäßrigen Lösung von Tetrabutylammoniumhydroxid werden mit 65 ml Dichlormethan vorgelegt. Dann gibt man tropfenweise 1.43 ml (15 mMol) Dimethylsulfat hinzu, läßt 1 Stunde bei Raumtemperatur rühren und wäscht nacheinander mit wenig Wasser und gesättigter Kochsalzlösung. Die wäßrigen Phasen werden mit Dichlormethan ausgeschüttelt und die vereinigten organischen Phasen über Natriumsulfat getrocknet, abfiltriert und eingeengt. Der Rückstand wird mit wenig Essigsäureethylester verrührt. Das Produkt wird mit Hexan ausgefällt und getrocknet. Man erhält 1.67 g (48.3% d.Th.) 4-Chlor-6-nitro-anthranilsäuremethylester vom Smp.114-116°.

603 mg (2.87 mMol) 3-Phenylpropylthioessigsäure in 6 ml Dichlormethan werden unter Stickstoff mit 0.3 ml (3.4 mMol) Oxalylchlorid versetzt, 2 Stunden unter Rückfluß gerührt und dann eingeengt Der Rückstand wird in 3 ml 1,2-Dichlorethan aufgenommen und zu einer Lösung von 550 mg (2.39 mMol) 4-Chlor-6-nitro-anthranilsäuremethylester in 6 ml 1,2-Dichlorethan getropft. Man läßt 2 Stunden bei 60° nachrühren, kühlt auf Raumtemperatur ab und dampft unter vermindertem Druck ein. Chromatographie an Kieselgel mit Hexan/Essigsäureethylester (4:1) als Laufmittel ergibt 0.57 g (56.4% d.Th.) 4-Chlor-6-nitro-N-(3-phenylpropylthio)acetyl-anthranilsäuremethylester als gelbes Öl.

### Beispiel 26:

2.2 g (5.31 mM) 4,6-Dichlor-N-[3-(pyrimidin-5-yl)Ipropylthio]acetyl-anthranilsäuremethylester werden in 25 ml Tetrahydrofuran bei 0° unter Stickstoff vorgelegt. Tropfenweise gibt man 10.62 ml einer 1-molaren Tetrahydrofuranlösung von Natrium-bis-trimethylsilylamid hinzu. Man läßt 2 Stunden bei 0° nachrühren. Das Reaktionsgemisch wird auf 50 ml 2N Salzsäure/Eis gegossen und abfiltriert. Der Rückstand wird in wenig Essigsäureethylester aufgenoommen und bis zur beginnenden Fällung mit Hexan versetzt. Der gebildete Niederschlag wird abgenutscht, mit Hexan gewaschen und getrocknet. Man erhält 1.75 g (86.2% d.Th) 5,7-Dichlor-4-hydroxy-3-[3-(pyrimidin-5-yl)propylthio)-2(1H)-chinolon als rote Kristalle vom Smp. 252-254°.

Das Ausgangsmaterial kann folgendermaßen hergestellt werden:

20g (0.174 Mol) 5-Brompyrimidin, 19.8 ml (0.35 Mol) Propargylalkohol,1.22 g (1.74 Mol) Bis-triphenylphosphinpalladium-II-chlorid, 31.3 ml (0.225 Mol) Triethylamin und 1.62 g (8.5 mMol) Kupfer-I-chlorid werden in 160 ml Acetonitril unter Rühren 1 Stunde zum Rückfluß erhitzt. Man läßt auf Raumtemperatur abkühlen und zieht da Acetonitril unter vermindertem Druck ab. Der Rückstand wird in Essigsäureethylester aufgenommen und nacheinander mit Wasser und gesättigter Kochsalzlösung gewaschen. Die wäßrigen Phasen werden vereinigt und mit Essigsäureethylester extrahiert. Die vereinigten organischen Phasen werden über Natriumsulfat getrocknet, abfiltriert und eingeengt. Chromatographie an Kieselgel mit Hexan/Essigsäureethylester (4:1) als Laufmittel ergibt 5.85 g (25.1% d.Th.) 3-(Pyrimidin-5-yl)propargylalkohol vom Smp. 85°.

5.8 g (43.2 mMol) 3-(Pyrimidin-5-yl)propargylalkohol werden mit 0.58 g 5%-iger Palladiumkohle in 60 ml Tetrahydrofuran bei Raumtemperatur und Normaldruck 30 Stunden hydriert. Das Reaktionsgemisch wird über ein Glasfaserfilter abgenutscht und eingeengt. Man erhält 4.12 g (69% d.Th.) 3-(Pyrimidin-5-yl)propanol als farbloses Öl.

Zu einer unter Stickstoff gerührten Lösung von 6.5 g (47 mMol) 3-(Pyrimidin-5-yl)propanol und 9 ml (64.6 mMol) Triethylamin in 100 ml Dichlormethan werden bei Raumtemperatur 9.87 g (51.7 mMol) Tosylchlorid in 50 ml Dichlormethan zugetropft. Man läßt 16 Stunden bei Raumtemperatur rühren, wäscht mit Wasser und gesättigter Kochsalzlösung, trocknet über Natriumsulfat, filtriert und dampft unter vermindertem Druck ein. Man erhält 14.42 g 3-(Pyrimidin-5-yl)propyltosylat, das ohne weitere Reinigung weiterverarbeitet werden kann.

2.36 g (54 mMol) einer 55%-igen Suspension von Natriumhydrid in Mineralöl werden bei 0° in 100 ml Tetrahydrofuran vorgelegt. Man tropft bei 0 bis 10° zunächst 4.93g (45 mMol) Thioglykolsäureethylester hinzu und nach 20 Minuten bei 0° eine Lösung von 14.4 g (47 mMol) 3-(Pyrimidin-5-yl)propyltosylat in 50 ml Tetrahydrofuran hinzu. Man läßt 20 Minuten am Rückfluß rühren, kühlt auf Raumtemperatur ab, nimmt in Essigsäureethylester auf, wäscht mit Wasser und gesättigter Kochsalzlösung, trocknet über Natriumsulfat filtriert klar und engt unter vermindertem Druck ein. Chromatographie an Kieselgel mit Hexan/Essigsäureethylester ergibt 7.82 g (69.2% d.Th.) 3-(Pyrimidin-5-yl)propylthioessigsäureethylester als braunes Öl.

7.8 g (32.5 mMol) 3-(Pyrimidin-5-yl)propylthioessigsäureethylester in 20 ml Methanol werden tropfenweise mit 20 ml (40 mMol) 2N Natronlauge versetzt. Man läßt 16 Stunden bei Raumtemperatur nachrühren, versetzt mit wenig Wasser, extrahiert mit Dichlormethan, trocknet über Natriumsulfat, saugt ab und engt das Filtrat ein. Man erhält 3.13 g (46% d.Th.) 3-(Pyrimidin-5-yl)propylthioessigsäu re als braunes Öl.

Eine Lösung von 3 g (14.1 mMol) (Pyrimidin-5-yl)propylthioessigsäure und 1.55 ml (14.1 mMol) 4-Methylmorpholin in 100 ml Dichlormethan werden bei 0° unter Stickstoff tropfenweise mit 1.83 ml (14.1 mMol) Chlorameisensäurebutylester versetzt. Man läßt 40 Minuten bei 0° rühren, gibt 3.1 g (14.1 mMol) 4,6-Dichloranthranilsäuremethylester hinzu und läßt 20 Stunden nachrühren. Das Reaktionsgemisch wird mit wenig Wasser und gesättigter Kochsalzlösung gewaschen, über Natriumsulfat getrocknet und eingedampft. Chromatographie an Kieselgel mit Hexan/Essigsäureethylester (2:1) als Laufmittel ergibt 2.39 g (40.9% d.Th.) 4,6-Dichlor-N-[3-(pyrimidin-5-yl)lpropylthio]acetyl-anthranilsäuremethylester als gelbes Öl.

### Beispiel 27:

1.88 g (4.5 mMol) von 4,6-Dichlor-N-[3-(2-thienyl)propylthioacetyl]-anthranilsäuremethylester werden unter Argon in 25 ml Tetrahydrofuran gelöst, bei 0 bis 5° portionsweise mit insgesamt 13.5 ml (13.5 mMol) einer 1-molaren Lösung von Natrium-bis-trimethylsilylamid in Tetrahydrofuran versetzt und 3 Stunden bei Raumtemperatur gerührt. Die hellbraune Lösung wird auf ein Gemisch von 40 ml 2N Salzsäure und Eiswasser gegossen, gründlich durchgerührt, abgenutscht und mit Eiswasser neutral gewaschen. Das Nutschgut wird unter vermindertem Druck 1 Stunde bei 40° getrocknet, in 100 ml Essigsäureethylester gelöst und mit 100 ml Hexan versetzt. Der gebildete Niederschlag wird abgenutscht, mit wenig Hexan gewaschen und bei 40° unter vermindertem Druck getrocknet. Nach Umkristallisieren aus Ethanol erhält man 1.36 g (78.2% d. Th.) 5,7-Dichlor-3-[3-(2-thienyl)propylthio)-4-hydroxy-2-(1H)-chinolon,Smp. 205-207°.

Das Ausgangsmaterial kann beispielsweise folgendermaßen hergestellt werden:

Durch Veresterung von 7.7 g (20 mMol) 3-(2-Thienyl)acrylsäure mit 50 ml Ethanol in Gegenwart von 2 ml konzentriertes Schwefelsäure erhält man 8.75 g (96% d. Th.) 3-(2-Thienyl)acrylsäureethylester als hellbraunes Öl.

Durch Hydrierung 1.82 g (10 mMol) 3-(2-Thienyl)acrylsäureethylester in 40 ml Ethanol unter Normaldruck und in Gegenwart von 0.9 g 5%-iger Palladiumkohle erhält man 1.71 g (92.8% d. Th.) 3-(2-Thienyl)propionsäureethylester als farbloses Öl.

Durch Reduktion von 8.3 g (45 mMol) 3-(2-Thienyl)propionsäureethylester mit 2.56 g (67.5 mMol) Litiumaluminiumhydrid in 150 ml Tetrahydrofuran erhält man 6.25 g (97.6% d. Th.) 3-(2-Thienyl)propanol als farblose Flüssigkeit.

Durch Bromierung von 6.2 g (43.6 mMol) 3-(2-Thienyl)propanol mit 9 ml (96 mMol) Phosphortribromid in 80 ml Tetrachlormethan erhält man 3.95 g (44.17% d. Th.) 3-(2-Thienyl)-propylbromid als hellgelbe Flüssigkeit.

995 mg (22.8 mMol) Nariumhydrid als 55%-ige Suspension in Mineralöl werden bei 0 bis 5° in 30 ml Tetrahydrofuran vorgelegt und tropfenweise mit 2.125 ml (19 mMol) Thioglykolsäureethylester versetzt. Man läßt 30 Minuten bei 0 bis 5° nachrühren, tropft unter Stickstoff bei 0 bis 5° 3.9 g (19 mMol) 3-(2-Thienyl)propylbromid hinzu, läßt auf Raumtemperatur erwärmen und 3 Stunden rühren. Dann kühlt man erneut auf 0 bis 5° ab, versetzt mit 200 ml Essigsäureethylester und 100 ml Eiswasser, trennt die organische Phase ab und schüttelt zweimal mit je 100 ml Essigsäureethylester nach. Die vereinigten organischen Phasen werden dreimal mit je 100 ml Wasser und dann mit gesättigter Kochsalzlösung gewaschen, über Natriumsulfat getrocknet, filtriert und bei 35° unter vermindertem Druck eingedampft und destilliert. Man erhält 3.755 g (80.2% d. Th.) 3-(2-Thienyl)propylthioessigsäu reethylester als hellgelbe Flüssigkeit.

Durch Verseifung von 3.745 g (15.3 mMol) 3-(2-Thienyl)propylthioessigsäureethylester mit 10 ml 2N Naronlauge in 30 ml Ethanol erhält 3.26 g (98.5% d. Th.) 3-(2-Thienyl)propylthioessigsäureethylester als hellgelbes Öl, durch Chlorierung von 3.24 g (15 mMol) desselben mit 2.84 ml (33 mMol) Oxalylchlorid in 35 ml Dichlormethan 3-(2-Thienyl)propylthioacetylchlorid als gelbes Öl und durch 16-stündige Umsetzung desselben mit 2.97 g (13.5 mMol) 4.6-Dichloranthranilsäuremethylester in Gegenwart von 2.3 ml (16.5 mMol) Triethylamin in 80 ml Dichlormethan bei Raumtemperatur, Verdünnen mit 200 ml Dichlormethan, Waschen mit Wasser (3x je 100 ml) und 100 ml gesättigter Kochsalzlösung, Trocknen über Natriumsulfat, Eindampfen unter vermindertem Druck und Chromatographie an Kieselgel mit Toluol als Laufmittel 1.97 g (34.9% d. Th. Gesamtausbeute) 4,6-Dichlor-N-[3-(2-thienyl)propylthioacetyl]-anthranilsäu remethylester als gelbliches Öl.

### Beispiel 28:

In analoger Weise wie in Beispiel 27 beschrieben erhält man ausgehend von 3-(3-Thienyl)acrylsäure über 4,6-Dichlor-N-[3-(3-thienyl)propylthioacetyl]-anthranilsäuremethylester 5,7-Dichlor-3-[3-(3-thienyl)propylthio)-4-hydroxy-2-(1H)-chinolon, Smp. 194-196°.

### Beispiel 29:

In analoger Weise wie in Beispiel 14 beschrieben erhält man durch Cyclisierung von 6-Chlor-N-(benzyloxyacetyl]-anthranilsäuremethylester mittels Natrium-N,N-bis(trimethylsilyl)amid in Tetrahydrofuran 7-Chlor-3-benzyloxy-4-hydroxy-2-(1H)-chinolon, Smp. 223-226°.

### Beispiel 30:

In analoger Weise wie in Beispiel 27 beschrieben erhält man ausgehend von 1,2,3,4-Tetrahydronaphthoesäure über 4,6-Dichlor-N-(1,2,3,4-tetrahydronaphth-2-ylmethylthioacetyl)-anthranilsäuremethylester 5,7-Dichlor-3-(1,2,3,4-tetrahydronaphth-2-ylmethylthio)-4-hydroxy-2-(1H)-chinolon, Smp. 213-215°.

### Beispiel 31:

In analoger Weise wie in Beispiel 27 beschrieben erhält man ausgehend von 1,4-Benzodioxan-2-ylmethanol über 4,6-Dichlor-N-(1,4-benzodioxan-2-ylmethylthioacetyl)-anthranilsäuremethylester 5,7-Dichlor-3-(1,4-benzodioxan-2-ylmethylthio)-4-hydroxy-2-(1H)-chinolon, Smp. 252-254°

### Beispiel 32:

In analoger Weise wie in Beispiel 27 beschrieben erhält man ausgehend von Chroman-3-carbonsäure über 4,6-Dichlor-N-(chroman-3-ylmethylthioacetyl)-anthranilsäuremethylester 5,7-Dichlor-3-(chroman-3-ylmethylthio)-4-hydroxy-2-(1H)-chinolon, Smp. 256-258°.

### Beispiel 33:

In analoger Weise wie in Beispiel 1 beschrieben erhält man durch Cyclisierung von 4,6-Dichlor-N-(2-phenyloxyethylthioacetyl)-anthranilsäuremethylester mittels Natrium-N,N-bis(trimethylsilyl)amid in Tetrahydrofuran 5,7-Dichlor-3-(2-phenyloxyethylthio)-4-hydroxy-2-(1 H)-chinolon, Smp. 229-231°.

### Beispiel 34:

In analoger Weise wie in den Beispielen 1, 7 bis 9 und 11 bis 15 beschrieben kann man ferner herstellen:
5,7-Dichlor-3-(3-phenylpropylthio)-4-hydroxy-2-(1H)-chinolon, Smp. 205-206°;
7-Chlor-3-heptylthio-4-hydroxy-2-(1H)-chinolon, Smp.155-157°;
7-Chlor-3-ylthio-4-hydroxy-2-(1H)-chinolon, Smp.296°;
7-Chlor-3-ethoxycarbonylmethlythio-4-hydroxy-2-(1H)-chinolon, Smp.194-197°;
7-Chlor-3-(N-methylcarbamoylmethyithio)-4-hydroxy-2-(1H)-chinolon, Smp. 253-255°;
7-Chlor-3-(3-*trans*-phenylprop-2-enylthio)-4-hydroxy-2-(1H)-chinolon , Smp. 209-211°;
7-Chlor-3-(3-*cis*-phenylprop-2-enylthio)-4-hydroxy-2-(1H)-chinolon;
3-Carboxymethyl-5,7-dichlor-4-hydroxy-2-(1H)-chinolon, Smp. über 300°;
3-Benzylthio-4-hydroxy-2(1H)-chinolon;
4-Hydroxy-3-(2-phenylethylthio)-2(1H)-chinolon;
4-Hydroxy-3-(3-phenylpropylthio)-2(1H)-chinolon;
4-Hydroxy-3-(4-phenylbutylthio)-2(1H)-chinolon;
3-(Carboxymeththio)-4-hydroxy-2(1H)-chinolon;
3-(3-Carboxypropylthio)-4-hydroxy-2(1H)-chinolon;
4-Hydroxy-3-(3-methoxycarbonylpropylthio)2(1H)-chinolon.

### Beispiel 35:

Tabletten, enthaltend je 50 mg 3-(3-Carboxypropylthio)-2(1H)-chinolon oder ein Salz, z.B. das Natriumsalz, davon können wie folgt hergestellt werden:

| Zusammensetzung (10000 Tabletten) | |
|---|---|
| Wirkstoff | 500.0 g |
| Laktose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdispers) | 20.0 g |
| Ethanol | q.s. |

Der Wirkstoff wird mit der Laktose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und preßt das Gemisch zu Tabletten von je 145,0 mg Gewicht und 50,0 mg Wirkstoffgehalt, die gewünschtenfalls mit Teilkerben zur feineren Anpassung der Dosierung versehen sein können.

### Beispiel 36:

Eine sterilfiltrierte wäßrige Gelatine-Lösung mit 20 % Cyclodextrinen als Lösungsvermittler, enthaltend je 3 mg 3-(3-Carboxypropylthio)-2(1 H)-chinolon oder ein Salz, z.B. das Natriumsalz, davon als Wirkstoff wird unter Erwärmen mit einer sterilen Gelatinelösung, welche als Konservierungsmittel Phenol enthält, unter aseptischen Bedingungen so vermischt, dass 1.0 ml Lösung die folgende Zusammensetzung hat:

| | |
|---|---|
| Wirkstoff | 3 mg |
| Gelatine | 150.0 mg |
| Phenol | 4.7 mg |
| dest. Wasser mit 20 % Cyclodextrinen als Lösungsvermittler | 1.0 ml |

### Beispiel 37:

Zur Herstellung einer sterilen Trockensubstanz zur Injektion, enthaltend je 5 mg 3-(3-Carboxypropylthio)-2(1 H)-chinolon oder ein Salz, z.B. das Natriumsalz, davon löst man 5 mg einer der in den vorausgehenden Beispielen genannten Verbindungen der Formel I als Wirkstoff in 1 ml einer wäßrigen Lösung mit 20 mg Mannit und 20 % Cyclodextrinen als Lösungsvermittler. Die Lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

### Beispiel 38:

Für die Herstellung von 10 000 Lacktabletten, enthaltend je 100 mg 3-(3-Carboxypropylthio)-2(1 H)-chinolon oder ein Salz, z.B. das Natriumsalz, davon können wie folgt hergestellt werden:

| | |
|---|---|
| Wirkstoff | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | q.s. |

Ein Gemisch von einer der in den vorausgehenden Beispielen genannten Verbindungen der Formel I als Wirkstoff, 50 g Maisstärke und der kolloidalen Kieselsäure wird mit Stärkekleister aus 250 g Maisstärke und 2.2 kg entmineralisiertem Wasser zu einer feuchten Masse verarbeitet. Diese wird durch ein Sieb von 3 mm Maschenweite getrieben und bei 45 ° während 30 Minuten. im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

### Beispiel 1339:

In analoger Weise wie in den Beispielen 35 bis 38 beschrieben kann man ferner pharmazeutische Präparate enthaltend eine andere Verbindung gemäß einem der Beispielen 1 bis 34 herstellen.

## Patentansprüche

1. Neue 3-Heteroaliphatyl- und 3-Hetero(aryl)aliphatyl-2(1H)-chinolonderivate der Formel I 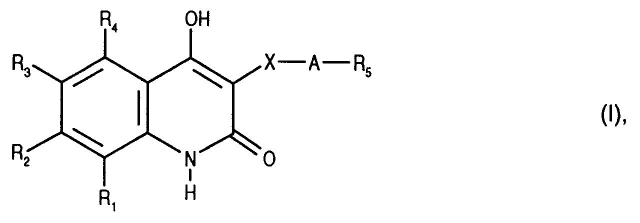 worin
die Reste R₁, R₂, R₃ und R₄ unabhängig voneinander Wasserstoff, aliphatische Kohlenwasserstoffreste, gegebenenfalls verethertes Hydroxy, gegebenenfalls verethertes und/oder oxidiertes Mercapto, unsubstituiertes oder aliphatisch substituiertes Amino, Nitro, gegebenenfalls verestertes oder amidiertes Carboxy, Cyano, gegebenenfalls amidiertes Sulfamoyl, Halogen oder Trifluormethyl bedeuten,
X Oxy oder gegebenenfalls oxidiertes Thio darstellt,
A für einen zweiwertigen aliphatischen Rest steht und
R₅ einen unsubstituierten oder durch aliphatische oder araliphatische Kohlenwasserstoffreste, gegebenenfalls verethertes Hydroxy, gegebenenfalls verethertes und/oder oxidiertes Mercapto, unsubstituiertes oder aliphatisch substituiertes Amino, aliphatisches Acyl, gegebenenfalls verestertes oder amidiertes Carboxy, Cyano, gegebenenfalls amidiertes Sulfamoyl, Halogen und/oder Trifluormethyl substituierten, gegebenenfalls partiell hydrierten Aryl- oder Hetroarylrest, gegebenenfalls. verethertes Hydroxymethyl, Cyano oder gegebenenfalls verestertes oder amidiertes Carboxy darstellt,
ihre Tautomeren und/oder Salze.

2. Verbindungen gemäß Anspruch 1 der Formel I, worin
die Reste R₁, R₂, R₃ und R₄ unabhängig voneinander Wasserstoff, aliphatische Kohlenwasserstoffreste, gegebenenfalls verethertes Hydroxy, gegebenenfalls verethertes und/oder oxidiertes Mercapto, unsubstituiertes oder aliphatisch substituiertes Amino, Nitro, gegebenenfalls verestertes oder amidiertes Carboxy, Cyano, gegebenenfalls amidiertes Sulfamoyl, Halogen oder Trifluormethyl bedeuten,
X Oxy oder gegebenenfalls oxidiertes Thio darstellt,
A für einen zweiwertigen aliphatischen Rest steht und
R₅ unsubstituiertes oder durch einen aliphatische oder araliphatische Kohlenwasserstoffreste, gegebenenfalls verethertes Hydroxy, gegebenenfalls verethertes und/oder oxidiertes Mercapto, unsubstituiertes oder aliphatisch substituiertes Amino, aliphatisches Acyl, gegebenenfalls verestertes oder amidiertes Carboxy, Cyano, gegebenenfalls amidiertes Sulfamoyl, Halogen und/oder Trifluormethyl substituiertes Aryl oder Hetroaryl, Cyano oder gegebenenfalls verestertes oder amidiertes Carboxy darstellt,
und ihre Tautomeren und/oder Salze.

3. Verbindungen gemäß Anspruch 1 Formel I, worin
die Reste R₁, R₂, R₃ und R₄ unabhängig voneinander Wasserstoff, Niederalkyl, Niederalkenyl, Niederalkinyl, Hydroxy, Niederalkoxy, Niederalkenyloxy, Niederalkinyloxy, Mercapto, Sulfo, Niederalkylthio, Niederalkansulfinyl, Niederalkansulfonyl, Niederalkensulfonyl, Amino, N-Mono- oder N,N-Diniederalkylamino, Nitro, Carboxy, Niederalkoxycarbonyl, unsubstituiertes oder durch Niederalkyl, Niederalkoxy, Hydroxy, Halogen und/oder Trifluormethyl substituiertes Phenylniederalkoxycarbonyl, Carbamoyl, N-Mono- oder N,N-Diniederalkylcarbamoyl, Cyano, Sulfamoyl, N-Mono- oder N,N-Diniederalkylsulfamoyl, Halogen oder Trifluormethyl bedeuten,
X Oxy, Thio, Sulfinyl oder Sulfonyl darstellt,
A für Niederalkylen, Niederalkenylen, Carboxyniederalkylen, Niederalkoxycarbonylniederalkylen, die Hydroxygruppe in höherer als der α-Stellung tragendes Hydroxyniederalkylen, die Niederalkoxygruppe in höherer als der a-Stellung tragendes Niederalkoxyniederalkylen oder die Niederalkenyloxygruppe in höherer als der α-Stellung tragendes Niederalkenyloxyniederalkylen steht und
R₅ einen unsubstituierten oder durch Niederalkyl, Niederalkenyl, Niederalkinyl, Phenylniederalkyl, Hydroxy, Niederalkoxy, Niederalkenyloxy, Niederalkinyloxy, Mercapto, Sulfo, Niederalkylthio, Niederalkansulfinyl, Niederalkansulfonyl, Niederalkensulfonyl Amino, N-Mono- oder N,N-Diniederalkylamino, Niederalkanoyl, Carboxy, Niederalkoxycarbonyl, unsubstituiertes oder durch Niederalkyl, Niederalkoxy, Hydroxy, Halogen und/oder Trifluormethyl substituiertes Phenylniederalkoxycarbonyl, Carbamoyl, N-Mono- oder N,N-Diniederalkylcarbamoyl, Cyano, Sulfamoyl, N-Mono- oder N,N-Diniederalkylsulfamoyl, Halogen und/oder Trifluormethyl mono-, di- oder trisubstituierten monocyclischen oder bicyclischen Arylrest, partiell hydrierten, vorzugsweise über ein gesättigtes C-Atom gebundenen, bicyclischen Arylrest, über ein C-Atom gebundenen, als Heteroatom(e) 1, 2, 3 oder 4 N-Atome oder 1 O- oder S-Atom aufweisenden monocyclischen oder bicyclischen Heteroarylrest oder partiell hydrierten, vorzugsweise über ein gesättigtes C-Atom gebundenen, 1 oder 2 O-oder S-Atome aufweisenden bicyclischen Heteroarylrest, Carboxy, Hydroxymethyl, Phenyloxymethyl, Niederalkoxycarbonyl, Carbamoyl, N-Mono- oder N,N-Diniederalkylcarbamoyl, jeweils unsubstituiertes oder im Phenylteil durch Niederalkyl, Niederalkoxy, Hydroxy, Halogen und/oder Trifluormethyl substituiertes N-Phenylcarbamoyl oder N-Phenyl-N-niederalkyl-carbamoyl oder Cyano darstellt,
und ihre Tautomeren und/oder Salze.

4. Verbindungen gemäß Anspruch 2 der Formel I, worin
die Reste R₁, R₂, R₃ und R₄ unabhängig voneinander Wasserstoff, C₁-C₄-Alkyl, Hydroxy, C₁-C₄-Alkoxy, Mercapto, Sulfo, C₁-C₄-Alkylthio, C₁-C₄-Alkansulfinyl, C₁-C₄-Alkansulfonyl, Amino, N-Mono- oder N,N-Diniederalkylamino, Nitro, Carboxy, C₁-C₄-Alkoxycarbonyl, unsubstituiertes oder durch C₁-C₄-Alkyl, C₁-C₄-Alkoxy, Hydroxy, Halogen der Atomnummer bis und mit 35 und/oder Trifluormethyl substituiertes Phenyl-C₁-C₄-alkoxycarbonyl, Carbamoyl, N-Mono- oder N,N-Di-C₁-C₄-alkylcarbamoyl, Cyano, Sulfamoyl, N-Mono- oder N,N-Di-C₁-C₄-alkylsulfamoyl, Halogen der Atomnummer bis und mit 35 oder Trifluormethyl bedeuten,
X Thio, Sulfinyl, Sulfonyl oder Oxy darstellt,
A für jeweils geradkettiges C₂-C₇-Alkylen, Carboxy-C₁-C₄-alkylen, C₁-C₄-Alkoxycarbonyl-C₁-C₄-alkylen, die Hydroxygruppe in höherer als der α:-Stellung tragendes Hydroxy-C₂-C₇-alkylen oder die Niederalkoxygruppe in höherer als der α:-Stellung tragendes C₁-C₄-Alkoxy-C₂-C₇-alkylen steht und
R₅ unsubstituiertes oder durch C₁-C₄-Alkyl, Hydroxy, C₁-C₄-Alkoxy, Mercapto, Sulfo, C₁-C₄-Alkylthio, C₁-C₄-Alkansulfinyl, C₁-C₄-Alkansulfonyl, Amino, N-Mono- oder N,N-Di-C₁-C₄-alkylamino, Nitro, C₁-C₄-Alkanoyl, Carboxy, C₁-C₄-Alkoxycarbonyl, unsubstituiertes oder durch C₁-C₄-Alkyl, C₁-C₄-Alkoxy, Hydroxy, Halogen der Atomnummer bis und mit 35, wie Chlor, und/oder Trifluormethyl substituiertes Phenyl-C₁-C₄-alkoxycarbonyl, Carbamoyl, N-Mono- oder N,N-Di-C₁-C₄-alkylcarbamoyl, Cyano, Sulfamoyl, N-Mono- oder N,N-Di-C₁-C₄-alkylsulfamoyl, Halogen der Atomnummer bis und mit 35 und/oder Trifluormethyl mono-, di- oder trisubstituiertes Phenyl, Carboxy, C₁-C₄-Alkoxycarbonyl, Carbamoyl, N-Mono- oder N,N-Di-C₁-C₄-alkylcarbamoyl oder Cyano darstellt,
ihre Tautomeren und/oder Salze.

5. Verbindungen gemäß Anspruch 1 der Formel I, worin
die Reste R₁, R₂, R₃ und R₄ unabhängig voneinander Wasserstoff, C₁-C₄-Alkyl, Hydroxy, C₁-C₄-Alkoxy, Mercapto, Sulfo, C₁-C₄-Alkylthio, C₁-C₄-Alkansulfinyl, C₁-C₄-Alkansulfonyl, Amino, N-Mono- oder N,N-Diniederalkylamino, Nitro, Carboxy, C₁-C₄-Alkoxycarbonyl, unsubstituiertes oder durch C₁-C₄-Alkyl, C₁-C₄-Alkoxy, Hydroxy, Halogen der Atomnummer bis und mit 35 und/oder Trifluormethyl substituiertes Phenyl-C₁-C₄-alkoxycarbonyl, wie Benzyloxycarbonyl oder α-Phenylethoxycarbonyl, Carbamoyl, N-Mono- oder N,N-Di-C₁-C₄-alkylcarbamoyl, Cyano, Sulfamoyl, N-Mono- oder N,N-Di-C₁-C₄-alkylsulfamoyl, Halogen der Atomnummer bis und mit 35 oder Trifluormethyl bedeuten,
X Thio, Sulfinyl, Sulfonyl oder in zweiter Linie Oxy darstellt,
A für jeweils geradkettiges C₂-C₇-Alkylen, C₂-C₇-Alkenylen, Carboxy-C₁-C₄-alkylen, Niederalkoxycarbonylniederalkylen, die Hydroxygruppe in höherer als der α-Stellung tragendes Hydroxyniederalkylen oder die Niederalkoxygruppe in höherer als der α-Stellung tragendes Niederalkoxyniederalkylen steht und
R₅ einen unsubstituierten oder durch C₁-C₄-Alkyl, Hydroxy, C₁-C₄-Alkoxy, Mercapto, Sulfo, C₁-C₄-Alkylthio, C₁-C₄-Alkansulfinyl, C₁-C₄-Alkansulfonyl, Amino, N-Mono- oder N,N-Di-C₁-C₄-alkylamino, Nitro, C₁-C₄-Alkanoyl, Carboxy, C₁-C₄-Alkoxycarbonyl, unsubstituiertes oder durch C₁-C₄-Alkyl, C₁-C₄-Alkoxy, Hydroxy, Halogen der Atomnummer bis und mit 35 und/oder Trifluormethyl substituiertes Phenyl-C₁-C₄-alkoxycarbonyl, Carbamoyl, N-Mono- oder N,N-Di-C₁-C₄-alkylcarbamoyl, Cyano, Sulfamoyl, N-Mono- oder N,N-Di-C₁-C₄-alkylsulfamoyl, Halogen der Atomnummer bis und mit 35 und/oder Trifluormethyl mono-, di- oder trisubstituierten Phenyl-, Naphthyl-, Indenyl-, 1,2,3,4-Tetrahydronaphth-1-yl-, 1,2,3,4-Tetrahydronaphth-2-yl-, Indan-1-yl-, Indan-2-yl-, Pyrrolyl-, Indolyl-, Furyl-, Benzofuranyl-, Thienyl-, Benzothienyl-, Imidazolyl-, Benzimidazolyl-, Oxazolyl-, Benzoxazolyl-, Thiazolyl-, Benzothiazolyl-, Tetrazolyl-, Pyridyl-, Chinolinyl-, Isochinolinyl-, Pyrimidinyl-, Chinazolinyl-, Pyridazinyl-, Cinnolinyl-, Pyrazinyl-, Chinoxalinyl-, Triazinyl-, Chroman-3-yl-, Chroman-4-yl- oder 1,4-Benzdioxan-2-ylrest, Carboxy, Hydroxymethyl, Phenyloxymethyl, C₁-C₄-Alkoxycarbonyl, Carbamoyl, N-Mono- oder N,N-Di-C₁-C₄-alkylcarbamoyl, jeweils unsubstituiertes oder im Phenylteil durch C₁-C₄-Alkyl, C₁-C₄-Alkoxy, Hydroxy, Halogen und/oder Trifluormethyl substituiertes N-Phenylcarbamoyl oder N-Phenyl-N-C₁-C₄-alkyl-carbamoyl oder Cyano darstellt,
und ihre Tautomeren und/oder Salze.

6. Verbindungen gemäß Anspruch 1 der Formel I, worin
R₁ und R₃ Wasserstoff bedeuten,
R₂ Halogen der Atomnummer bis und mit 35, wie Chlor, ist,
R₄, Wasserstoff, Halogen der Atomnummer bis und mit 35, C₁-C₄-Alkyl oder Nitro bedeutet,
X Thio darstellt, A für geradkettiges C₁-C₄-Alkylen steht und
R₅ einen unsubstituierten oder durch Halogen der Atomnummer bis und mit 35, Cyano, Carboxy, C₁-C₄-Alkoxycarbonyl, N-C₁-C₄-Alkylcarbamoyl, N-Phenyl-N-C₁-C₄-alkylcarbamoyl, Hydroxy, C₁-C₄-Alkanoyl und/oder C₁-C₄-Alkyl mono- oder disubstituiertes Phenyl-, Pyrimidinyl-, Chromanyl-, 1,4-Benzodioxanyl-, Thienyl- oder 1,2,3,4-Tetrahydronaphth-2-ylrest, Carboxy, C₁-C₄-Alkoxycarbonyl oder N-C₁-C₄-Alkylcarbamoyl darstellt,
und ihre Tautomeren und/oder Salze.

7. Verbindungen gemäß Anspruch 2 der Formel I, worin
R₁ und R₃ Wasserstoff bedeuten,
R₂ Halogen der Atomnummer bis und mit 35 ist,
R₄ Wasserstoff oder Halogen der Atomnummer bis und mit 35 bedeutet,
X Thio darstellt,
A für geradkettiges C₁-C₄-Alkylen steht und
R₅ unsubstituiertes oder durch Halogen der Atomnummer bis und mit 35, Cyano, Carboxy, C₁-C₄-Alkoxycarbonyl, Hydroxy, C₁-C₄-Alkanoyl und/oder C₁-C₄-Alkyl mono- oder disubstituiertes Phenyl, Carboxy oder C₁-C₄-Alkoxycarbonyl darstellt,
und ihre Tautomeren und/oder Salze.

8. Verbindungen gemäß Anspruch 1 der Formel I, worin
R₁ und R₃ Wasserstoff bedeutet,
R₂ Halogen der Atomnummer bis und mit 35 ist,
R₄ Wasserstoff, C₁-C₄-Alkyl, Halogen der Atomnummer bis und mit 35 oder Nitro bedeutet,
X Thio darstellt,
A für geradkettiges C₁-C₄-Alkylen steht und
R₅ unsubstituiertes oder durch Halogen der Atomnummer bis und mit 35, C₁-C₄-Alkoxy oder Carboxy substituiertes Phenyl, Thienyl, 1,2,3,4-Tetrahydronaphth-2-yl, Pyrimidin-5-yl, Chroman-2-yl, 1,4-Benzodioxan-3-yl, Carboxy oder C₁-C₄-Alkoxycarbonyl darstellt,
und ihre Tautomeren und/oder Salze.

9. Verbindungen gemäß Anspruch 2 der Formel I, worin
R₁ und R₃ Wasserstoff bedeutet,
R₂ Halogen der Atomnummer bis und mit 35 ist,
R₄ Wasserstoff, C₁-C₄-Alkyl oder Halogen der Atomnummer bis und mit 35 bedeutet,
X Thio darstellt,
A für geradkettiges C₁-C₄-Alkylen steht
und R₅ unsubstituiertes oder durch Halogen der Atomnummer bis und mit 35 substituiertes Phenyl, Carboxy oder C₁-C₄-Alkoxycarbonyl darstellt,
und ihre Tautomeren und/oder Salze.

10. 7-Chlor-4-hydroxy-3-(3-phenylpropylthio)-2(1H)-chinolon,
18.7-Chlor-4-hydroxy-3-(methoxycarbonylmethylthio)-2(1H)-chinolon.
7-Chlor-4-hydroxy-3-(carboxymethylthio)-2(1H)-chinolon,
7-Chlor-4-hydroxy-3-(3-methoxycarbonylpropylthio)2(1H)-chinolon,
3-(3-Carboxypropylthio)-7-chlor-4-hydroxy-2(1H)-chinolon,
7-Chlor-3-(4-phenylbutylthio)4-hydroxy-2-(1H)-chinolon,
7-Chlor-3-(Benzylthio)-4-hydroxy-2-(1H)-chinolon,
7-Chlor-3-(benzylsulfinyl)-4-hydroxy-2-(1H)-chinolon oder
5,7-Dichlor-3-(3-phenylpropylthio)-4-hydroxy-2-(1H)-chinolon oder jeweils ein Salz davon.

11. 7-Chlor-5-ethyl-4-hydroxy-3-(3-phenylpropylthio)-2(1H)-chinolon,
7-Chlor-4-hydroxy-3-(2-phenethylhio)-2(1H)-chinolon,
7-Chlor-5-(hex-1-enyl)-4-hydroxy-3-(3-phenylpropylthio)-2(1H)-chinolon,
5,7-Dichlor-4-hydroxy-3-[3-(4-methoxycarbonylphenyl)propylthio]-2(1H)-chinolon,
5,7-Dichlor-4-hydroxy-3-[3-(4-carboxyphenyl)propylthio]-2(1H)-chinolon,
5,7-Dichlor-4-hydroxy-3-[3-(3-methoxycarbonylphenyl)propylthio]-2(1H)-chinolon,
5,7-Dichlor-4-hydroxy-3-[3-(3-carboxyphenyl)propylthio]-2(1H)-chinolon,
5,7-Dichlor-4-hydroxy-3-(carboxymethylthio)-2(1 H)-chinolon,
7-Chlor-4-hydroxy-3-(1-carboxy-3-phenyl-propylthio)-2(1H)-chinolon,
7-Chlor-4-hydroxy-3-(2-hydroxy-3-phenyl-propylthio)-2(1H)-chinolon,
7-Chlor-4-hydroxy-3-(2-hydroxyethylthio)-2(1H)-chinolon,
7-Chlor-3-(benzylsulfonyl)-4-hydroxy-2-(1H)-chinolon,
7-Chlor-3-(carboxymethansulfonyl)-4-hydroxy-2-(1H)-chinolon,
5,7-Dichlor-3-[3-(4-methoxyphenyl)propylthio)-4-hydroxy-2-(1H)-chinolon,
5,7-Dichlor-3-[3-(2-fluorphenyl)propylthio)-4-hydroxy-2-(1H)-chinolon,
5,7-Dichlor-3-(benzylthio)-4-hydroxy-2-(1H)-chinolon,
5,7-Dichlor-3-[3-(4-fluorphenyl)propylthio)-4-hydroxy-2-(1H)-chinolon,
7-Chlor-5-nitro-4-hydroxy-3-(3-phenylpropylthio)-2(1H)-chinolon,
5,7-Dichlor-4-hydroxy-3-[3-(pyrimidin-5-yl)propylthio)-2(1H)-chinolon,
5,7-Dichlor-3-[3-(2-thienyl)propylthio)-4-hydroxy-2-(1H)-chinolon,
5,7-Dichlor-3-[3-(thien-3-yl)propylthio)-4-hydroxy-2-(1H)-chinolon,
7-Chlor-3-benzyloxy-4-hydroxy-2-(1H)-chinolon,
5,7-Dichlor-3-(1,2,3,4-tetrahydronaphth-2-ylmethylthio)-4-hydroxy-2-(1H)-chinolon,
5,7-Dichlor-3-(1,4-benzodioxan-2-ylmethylthio)-4-hydroxy-2-(1H)-chinolon,
5,7-Dichlor-3-(chroman-3-ylmethylthio)-4-hydroxy-2-(1H)-chinolon oder
5,7-Dichlor-3-(2-phenyloxyethylthio)-4-hydroxy-2-(1H)-chinolon oder jeweils ein Salz davon.

12. Eine Verbindung gemäß einem der Ansprüche 1 bis 11 zur Behandlung des menschlichen oder tierischen Körpers.

13. Pharmazeutische Präparate, enhaltend eine Verbindung gemäß einem der Ansprüche 1, 3, 5, 6, 8, 11 und 12 oder ein pharmazeutisch verwendbares Salz davon neben üblichen pharmazeutischen Hilfs- und Trägerstoffen.

14. Pharmazeutische Präparate, enhaltend eine Verbindung gemäß einem der Ansprüche 1 Ansprüche 2, 4, 7, 9, und 10 oder ein pharmazeutisch verwendbares Salz davon neben üblichen pharmazeutischen Hilfs- und Trägerstoffen.

15. Verfahren zur Herstellung von neuen 3-Heteroaliphatyl- und 3-Hetero(aryl)aliphatyl-2(1H)-chinolonderivaten der Formel I 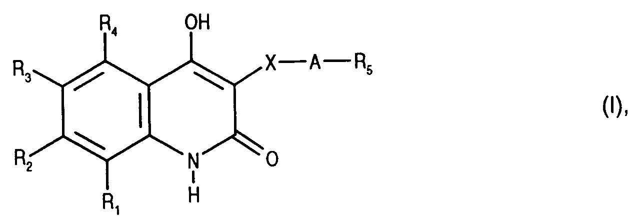 worin
die Reste R₁, R₂, R₃ und R₄ unabhängig voneinander Wasserstoff, aliphatische Kohlenwasserstoffreste, gegebenenfalls verethertes Hydroxy, gegebenenfalls verethertes und/oder oxidiertes Mercapto, unsubstituiertes oder aliphatisch substituiertes Amino, Nitro, gegebenenfalls verestertes oder amidiertes Carboxy, Cyano, gegebenenfalls amidiertes Sulfamoyl, Halogen oder Trifluormethyl bedeuten,
X Oxy oder gegebenenfalls oxidiertes Thio darstellt,
A für einen zweiwertigen aliphatischen Rest steht und
R₅ einen unsubstituierten oder durch aliphatische oder araliphatische Kohlenwasserstoffreste, gegebenenfalls verethertes Hydroxy, gegebenenfalls verethertes und/oder oxidiertes Mercapto, unsubstituiertes oder aliphatisch substituiertes Amino, aliphatisches Acyl, gegebenenfalls verestertes oder amidiertes Carboxy, Cyano, gegebenenfalls amidiertes Sulfamoyl, Halogen und/oder Trifluormethyl substituierten, gegebenenfalls partiell hydrierten Aryl- oder Hetroarylrest, gegebenenfalls. verethertes Hydroxymethyl, Cyano oder gegebenenfalls verestertes oder amidiertes Carboxy darstellt,
und ihrer Tautomeren und/oder Salze, dadurch gekennzeichnet, dass man eine Verbindung der Formel II 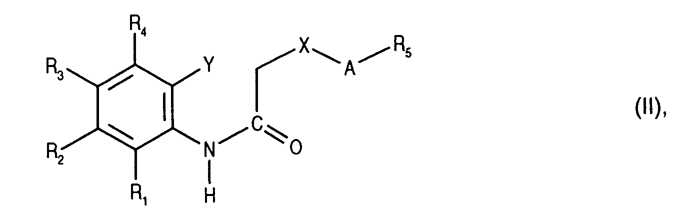 worin
Y eine reaktionsfähige Carboxyfunktion bedeutet,
intramolekular cyclisiert und gewünschtenfalls eine erhaltene Verbindung in eine andere Verbindung der Formel I überführt, ein verfahrensgemäß erhältliches Isomerengemisch in die Komponenten auftrennt und das jeweils bevorzugte Isomere abtrennt und/oder eine verfahrensgemäß erhältliche freie Verbindung in ein Salz oder eine verfahrensgemäß erhältliches Salz in die entsprechende freie Verbindung überführt.

16. Verwendung einer Verbindung gemäß einem der Ansprüche 1 bis 12 zur Herstellung eines für die Behandlung von pathologischen Zuständen, die auf Glycin-antagonistische Blockierung von NMDA-sensitiven Rezeptoren ansprechen, bestimmten Arzneimittels.
